# EUROPEAN PATENT APPLICATION

(11) **EP 4 729 072 A1**
(43) Date of publication of application: **22.04.2026**
(21) Application number: 25204752.7
(22) Date of filing: 25.09.2025
(51) Int. Cl.: A61K 47/54

(54) **GALNAC MONOMER COMPRISING RIBOSE RING OR DERIVATIVE STRUCTURE THEREOF AND USE THEREOF IN LIVER-TARGETED DELIVERY OF SMALL NUCLEIC ACID DRUGS**

(30) Priority: 26.09.2024 CN 202411356805
(71) Applicant: Beijing Youcare Kechuang Pharmaceutical Technology Co., Ltd., Daxing District, Beijing 100176 (CN)
(72) Inventor: HUANG, Zeao, China, 100176 (CN); SONG, Gengshen, Beijing, 100176 (CN); TIAN, Zhikang, Beijing, 100176 (CN); YANG, Shuo, China, 100176 (CN); WU, Yucheng, Beijing, 100176 (CN); FU, Rui, Beijing, 100176 (CN)
(74) Representative: Dehns

(57) **Abstract**

The present disclosure provides a GalNAc monomer comprising a ribose ring or a derivative structure thereof and a use thereof in liver-targeted delivery of small nucleic acid drugs. Specifically provided is a compound of formula (I) or a pharmaceutically acceptable salt thereof. The GalNAc-conjugated oligonucleotide prepared from the GalNAc compound provided by the present disclosure can achieve efficient liver-targeted delivery and enhance drug efficacy.

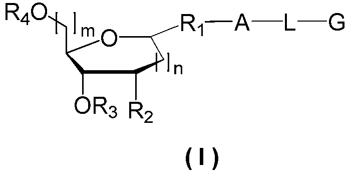

## Description

### TECHNICAL FIELD

The present application relates to the field of biomedicine, specifically to a GalNAc compound with a ribose ring structure, and a GalNAc-conjugated oligonucleotide prepared from the compound, which enables efficient liver-targeted delivery.

### BACKGROUND

Nucleic acid drugs, particularly oligonucleotide drugs, have been widely used due to their simple synthesis and high activity. Oligonucleotide drugs typically include antisense oligonucleotides (ASOs), small interfering RNAs (siRNAs), microRNAs (miRNAs), nucleic acid aptamers, and the like.

Oligonucleotides are short DNA or RNA molecules or oligomers. Oligonucleotides are readily bind to their respective complementary oligonucleotides, DNA, or RNA in a sequence-specific manner to form duplexes or, less commonly, higher-order hybrids. This fundamental property enables oligonucleotides to have broad applications in genetic testing, targeted gene therapy research, and medicine. These small nucleic acid fragments can be manufactured as single-stranded molecules with any specified sequence. In nature, oligonucleotides are often small RNA molecules that play a role in the regulation of gene expression, or they are degradation intermediates derived from the breakdown of larger nucleic acid molecules.

RNA interference is a natural defense mechanism against exogenous genes. siRNA can knock out target genes by recognizing specific sequences and degrading the target mRNA.

N-Acetylgalactosamine (GalNAc) is a ligand that binds to the asialoglycoprotein receptor (ASGPR) on the liver surface. The asialoglycoprotein receptor is an endocytic receptor specifically expressed on the surface of hepatocytes. In recent years, utilizing GalNAc, a high-affinity ligand of ASGPR, as a targeting molecule has achieved certain progress in the liver-targeted delivery of nucleic acid drugs. For example, Alnylam^{®} Pharmaceuticals, Inc. reported that siRNA based on GalNAc conjugation technology exerted gene silencing activity in mice (Nair JK, et al. J. Am. Chem. Soc. 2014, 136, 16958). The study reported that the conjugate of GalNAc and siRNA exhibited good delivery activity in both *in vivo* and *in vitro* experiments. Through *in vivo* experiments in mice via subcutaneous administration, the ED₅₀ of a single dose was determined to be 1 mg/kg, with a single injection volume of less than 1 mL. In long-term dosing experiments, stable interference activity lasting up to 9 months can be achieved with subcutaneous injection once a week. Studies have found that tetra-antennary and tri-antennary GalNAc compounds exhibit significantly higher affinity for ASGPR compared to di-antennary and mono-antennary GalNAc compounds.

GalNAc compounds with different structures demonstrate substantial variations in nucleic acid delivery efficiency. To enhance the delivery efficiency of liver-targeted drugs, such as AGT inhibitors, anti-chronic hepatitis B infection drugs, and lipid-lowering drugs, there is a need in the field to develop novel GalNAc compounds.

### BRIEF SUMMARY OF THE INVENTION

In order to solve the above technical problems, the present disclosure provides a GalNAc monomer comprising a ribose ring or a derivative structure thereof and a use thereof in liver-targeted delivery of small nucleic acid drugs. The GalNAc compound provided by the present disclosure can be used to prepare GalNAc-conjugated oligonucleotides, significantly improving delivery efficiency and significantly inhibiting AGT gene expression.

The present disclosure provides a compound of formula (I) or a pharmaceutically acceptable salt thereof: 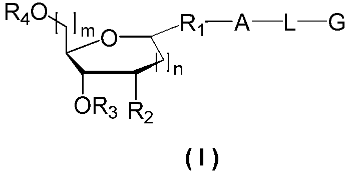 wherein
R₁ is O or S;
R₂ is H, C₁₋₆ alkyl, C₁₋₆ alkoxy, or halogen;
R₃ is H, a hydroxyl protecting group, a phosphorus-containing reactive group, - CO(CH₂)ₓCONH-○, or -CO(CH₂)ₓCOOH, wherein x is an integer from 1 to 10, ○ is controlled pore glass or polystyrene;
R₄ is H or a hydroxyl protecting group;
A is -(CH₂)ₐ-, -(CH₂CH₂OCH₂CH₂)_{b}-, or -(CH₂OCH₂)_{c}-, wherein a is an integer from 1 to 10; b is an integer from 1 to 5; c is an integer from 1 to 7;
L is -CONH- or -NHCO-;
G is 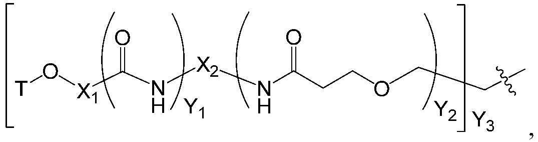
wherein
T is the following group in which all hydroxyl groups are protected with acyl groups: *N*-acetyl-galactosamine*,* galactose, galactosamine, *N*-formal-galactosamine, *N*-propionyl-galactosamine, or *N*-butyryl-galactosamine;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
Y₁ is 0 or 1;
Y₂ is 0, 1, or 2;
Y₃ is 1, 2, or 3;
m is an integer from 0 to 4;
n is an integer from 0 to 4.

In one embodiment, in the compound of formula (I) or the pharmaceutically acceptable salt thereof, the definitions of certain groups may be as described below, while the definitions of other groups may be as described in any one of the above embodiments (hereinafter referred to as "in one embodiment"):

In one embodiment, the C₁₋₆ alkyl is methyl, ethyl, *n*-propyl, isopropyl, *n*-butyl, isobutyl, or *tert*-butyl.

In one embodiment, the C₁₋₆ alkoxy is methoxy, ethoxy, *n*-propoxy, isopropoxy, *n-*butoxy, isobutoxy, or *tert*-butoxy.

In one embodiment, the halogen is fluorine, chlorine, or bromine.

In one embodiment, A is -CH₂CH₂OCH₂CH₂-, -(CH₂)₂-, -(CH₂)₃-, -(CH₂)₄-, or - (CH₂)₅-.

In one embodiment, R₁ is O.

In one embodiment, R₁ is in *α* configuration or β configuration, such as α configuration.

In one embodiment, R₂ is C₁₋₆ alkoxy, such as -OCH₃.

In one embodiment, in R₃, the hydroxyl protecting group is acyl, silyl, trityl, 4-methoxytrityl, or 4,4'-dimethoxytrityl, preferably acetyl, 1,1,3,3-tetraisopropyldisiloxanyl, *tert*-butyldimethylsilyl, dimethylphenylsilyl, or 4,4'-dimethoxytrityl.

In one embodiment, R₃ is a phosphorus-containing reactive group, such as

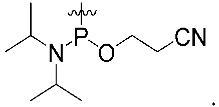

In one embodiment, R₃ is a phosphorus-containing reactive group or - CO(CH₂)ₓCONH-○, wherein ○ is controlled pore glass **(CPG)** or polystyrene, preferably, x is 2.

In one embodiment, R₃ is -CO(CH₂)₂CONH-○, wherein ○ is controlled pore glass (CPG).

In one embodiment, R₃ is -CO(CH₂)₂COOH.

In one embodiment, R₄ is a hydroxyl protecting group, preferably trityl, 4-methoxytrityl, or 4,4'-dimethoxytrityl, and more preferably 4,4'-dimethoxytrityl.

In one embodiment, T is *N*-acetyl-galactosamine in which all hydroxyl groups are protected with acyl groups, wherein the acyl group may be acetyl or benzoyl, such as acetyl.

In one embodiment, X₁ is -(CH₂)_{f}-, wherein f is preferably 1.

In one embodiment, a is an integer from 2 to 7.

In one embodiment, b is an integer from 1 to 3.

In one embodiment, g is 2.

In one embodiment, Y₁ is 0.

In one embodiment, Y₂ is 0.

In one embodiment, Y₃ is 1.

In one embodiment, m is 0, 1, or 2, such as 1.

In one embodiment, n is 0, 1, or 2, such as 0.

In one embodiment, L is -NHCO-.

In one embodiment, G is

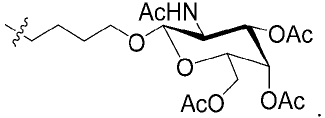

In one embodiment, the compound of formula (I) is a compound of formula I-1 below; 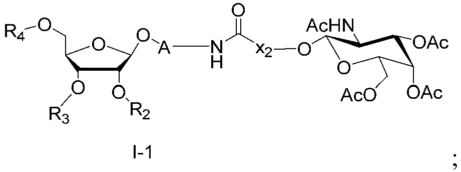 wherein R₂, R₃, R₄, A, and X₂ are independently as described in any one of the embodiments of the present disclosure.

In one embodiment, the compound of formula (I) is any one of the following compounds:

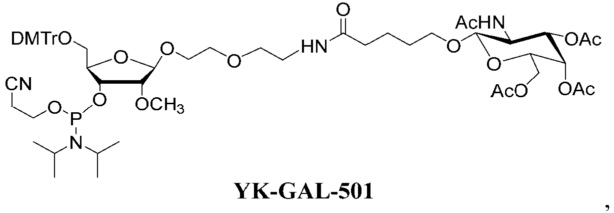

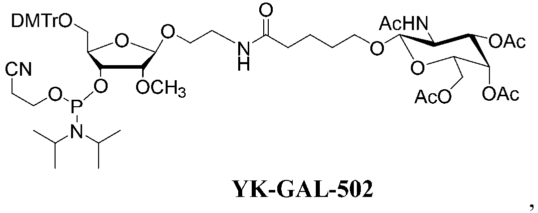

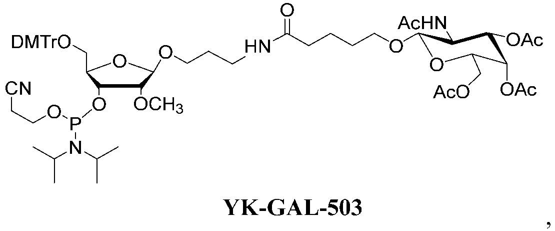

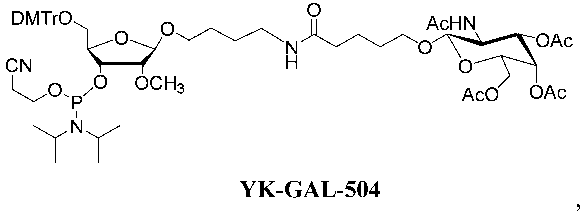

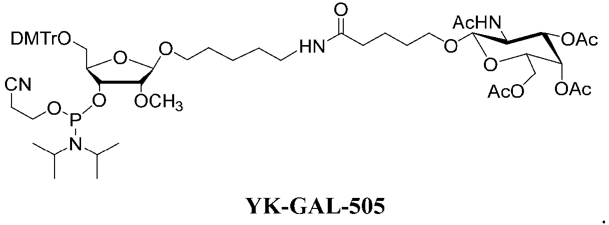

In one embodiment, the compound of formula (I) is any one of the following compounds:

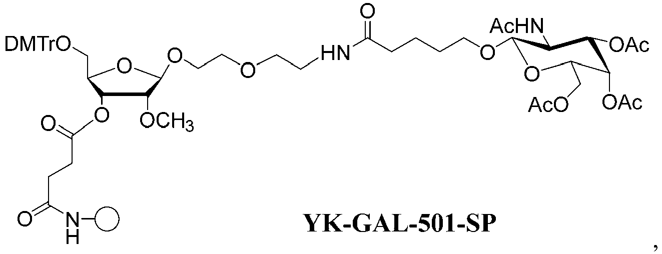

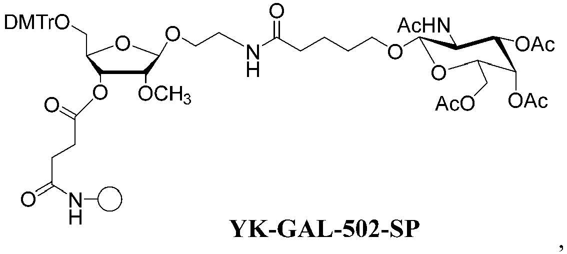

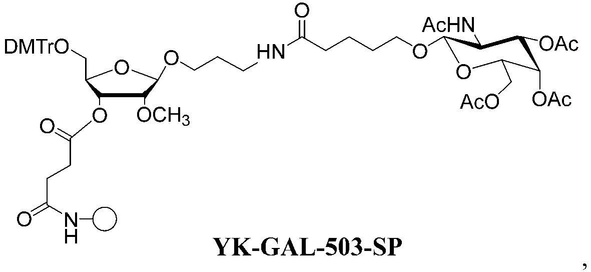

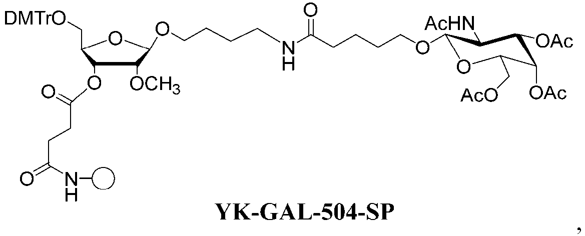

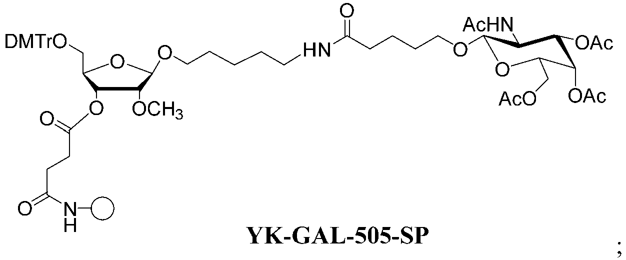

○ is controlled pore glass **(CPG).**

In one embodiment, the compound of formula (I) or the pharmaceutically acceptable salt thereof is capable of binding to an asialoglycoprotein receptor (ASGPR).

The present disclosure provides a use of the compound of formula (I) or the pharmaceutically acceptable salt thereof as an intermediate in the manufacture of a GalNAc-conjugated oligonucleotide drug.

The present disclosure provides a conjugate comprising an oligonucleotide and a GalNAc moiety, wherein the oligonucleotide and GalNAc are linked via a phosphoester group or a phosphorothioate group (linked via R₃₋₁ or R₄₋₁); the GalNAc moiety is formed by one or more GalNAc molecules linked via a phosphoester group or a phosphorothioate group; wherein the GalNAc molecule is the compound of formula (I) or the pharmaceutically acceptable salt thereof; 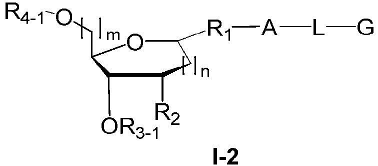
wherein R₃₋₁ is a linking bond, and R₄ is H;
alternatively, R₄₋₁ is a linking bond, and R₃₋₁ is H;
G is 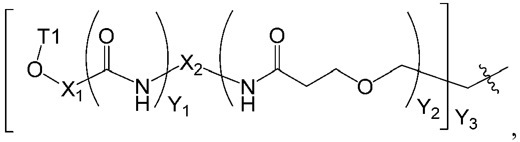
T1 is *N*-acetyl-galactosamine, galactose, galactosamine, *N*-formal-galactosamine, *N-*propionyl-galactosamine, or *N*-butyryl-galactosamine, preferably *N*-acetyl-galactosamine;
Y₃, Y₂, X₂, Y₁, X₁, m, n, R₂, R₁, A, and L are as defined in any one of the embodiments of the present disclosure.

In one embodiment, in the conjugate, the oligonucleotide may comprise a non-thio oligonucleotide and a thio oligonucleotide.

In one embodiment, in the conjugate, the non-thio oligonucleotide and the GalNAc moiety may be linked via a phosphoester bond.

In one embodiment, in the conjugate, the thio oligonucleotide and the GalNAc moiety may be linked via a phosphorothioate bond.

In one embodiment, the conjugate preferably has the following structure: 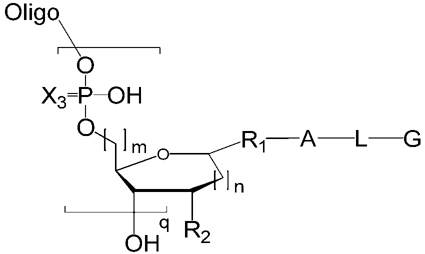 or 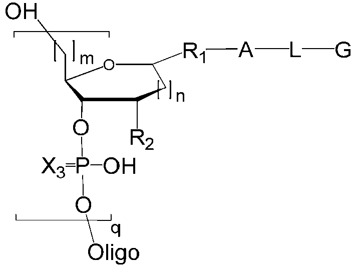
wherein Oligo represents an oligonucleotide, X₃ is O or S, and q is 1, 2, or 3;
R₁, R₂, A, L, G, m, and n are independently as described in any one of the embodiments of the present disclosure.

In one embodiment, in the conjugate, the oligonucleotide preferably comprises small interfering nucleotide (siRNA), DNA, microRNA (miRNA), small activating RNA (saRNA), small guide RNA (sgRNA), transfer RNA (tRNA), antisense nucleotide (ASO), or aptamer (Aptamer), preferably antisense nucleotide (ASO) or small interfering nucleotide (siRNA).

In one embodiment, in the conjugate, each nucleotide in the antisense nucleotide (ASO) or small interfering nucleotide (siRNA) is preferably independently a modified or unmodified nucleotide.

In one embodiment, the conjugate preferably has the following structure:

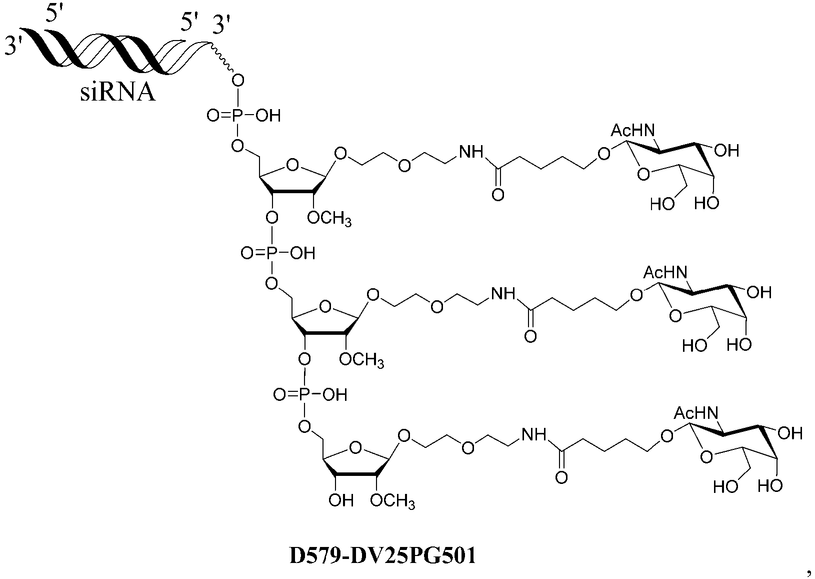

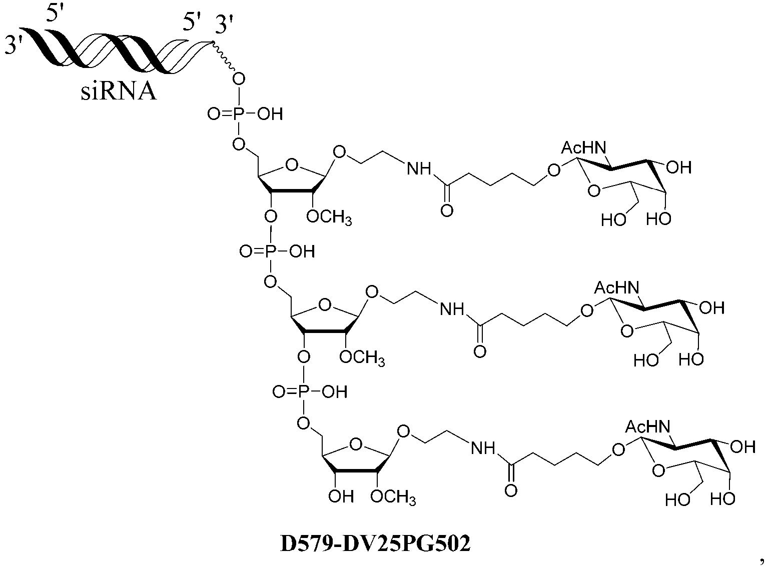

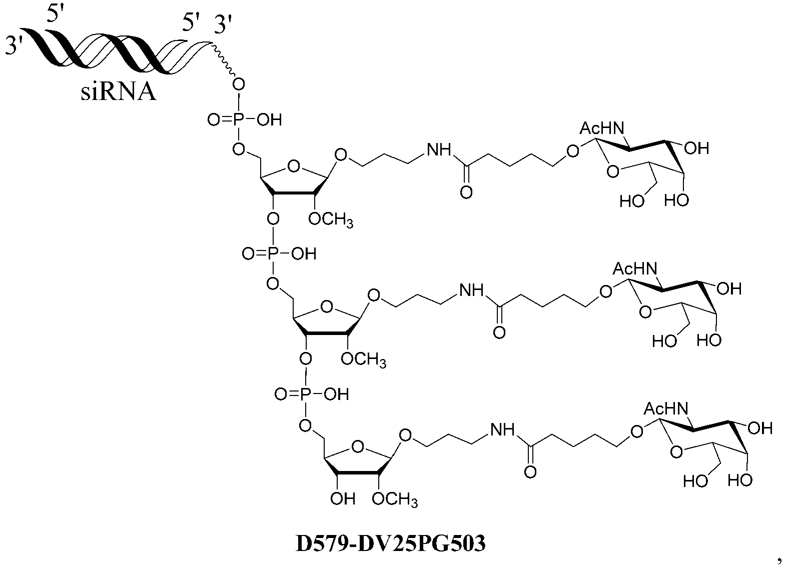

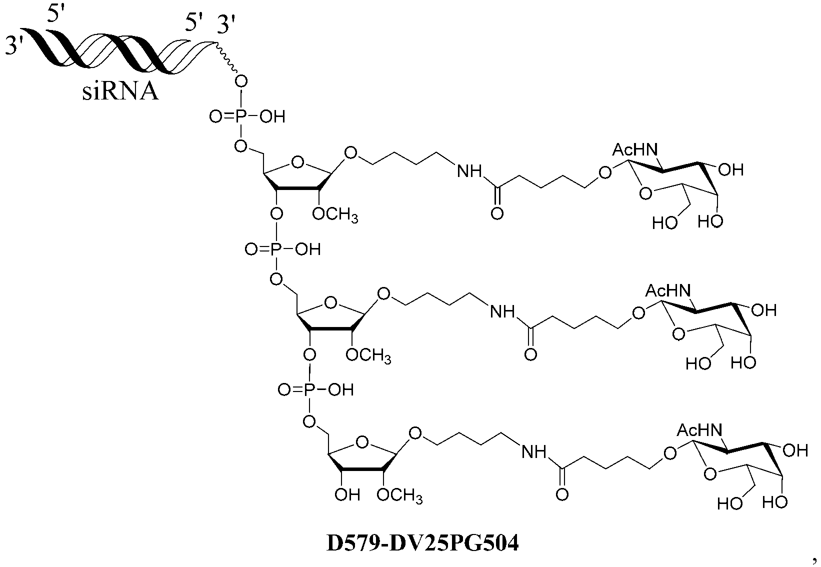

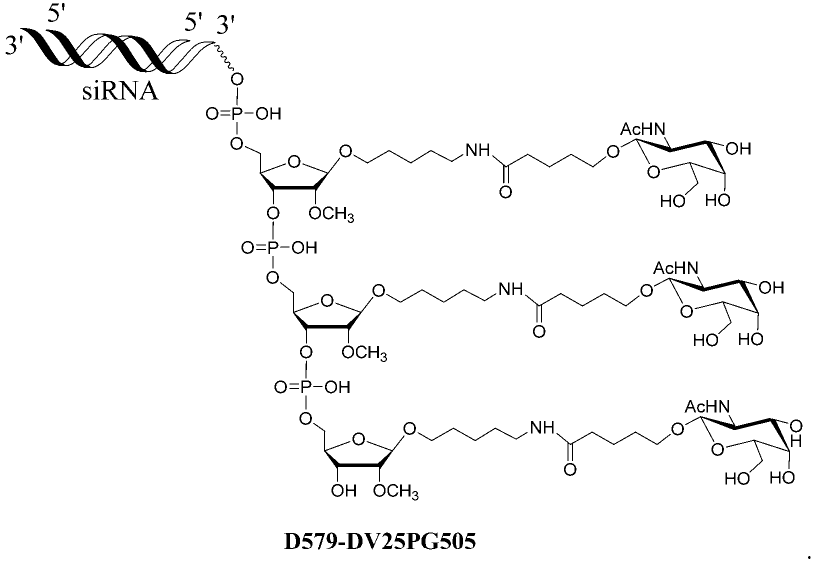

In the conjugate, the oligonucleotide modulates the expression of a target gene.

The present disclosure provides a pharmaceutical composition comprising the conjugate according to any one of the embodiments of the present disclosure and at least one pharmaceutically acceptable excipient.

The present disclosure provides a use of the conjugate according to any one of the embodiments of the present disclosure or a pharmaceutically acceptable salt thereof, or the pharmaceutical composition according to any one of the embodiments of the present disclosure, in the manufacture of a medicament for treating and/or preventing a pathological condition or disease caused by expression of a specific gene in liver tissue or in a virus; optionally, the specific gene is selected from the group consisting of a hepatitis B virus (HBV) gene, a proprotein convertase subtilisin/kexin type 9 (PCSK9) gene, a coagulation factor gene, a lipoprotein(a) gene, an angiopoietin-like protein 3 gene, an angiotensinogen gene, an apolipoprotein C3 gene, and the like. Preferably, the disease is selected from the group consisting of chronic liver disease, hepatitis, liver fibrosis, liver proliferative disease, and cardiovascular and cerebrovascular diseases; optionally, the cardiovascular and cerebrovascular diseases are hypercholesterolemia, hypertriglyceridemia, atherosclerosis, or coagulation dysfunction.

The present disclosure provides a method for inhibiting the expression of a specific gene in a hepatocyte, wherein the method comprises administering a therapeutically effective amount of the conjugate according to any one of the above embodiments to an individual in need thereof; preferably, the method comprises contacting the hepatocyte with an effective amount of the conjugate according to any one of the above embodiments; optionally, the specific gene is selected from the group consisting of a proprotein convertase subtilisin/kexin type 9 gene (PCSK9), a hepatitis B virus gene, an apolipoprotein(a) gene, a coagulation factor XI gene, an angiopoietin-like protein 3 gene, an angiotensinogen gene, and an apolipoprotein C3 gene.

The present disclosure provides a kit comprising the conjugate according to any one of the embodiments of the present disclosure.

### Term explanation:

The term "pharmaceutically acceptable" refers to being relatively non-toxic, safe, and suitable for patient use.

The term "pharmaceutically acceptable salt" refers to a salt obtained by reacting a compound with a pharmaceutically acceptable acid or base. When the compound contains a relatively acidic functional group, a base addition salt may be obtained by contacting the compound with a sufficient amount of a pharmaceutically acceptable base in a suitable inert solvent. When the compound contains a relatively basic functional group, an acid addition salt may be obtained by contacting the compound with a sufficient amount of a pharmaceutically acceptable acid in a suitable inert solvent. For details, refer to Handbook of Pharmaceutical Salts: Properties, Selection, and Use (P. Heinrich Stahl, Camille G. Wermuth, 2011, 2nd Revised Edition).

The term "acyl" refers to R-CO-, wherein R is C₁₋₆ alkyl, C₆₋₁₄ aryl, substituted C₁₋₆ alkyl, or substituted C₆₋₁₄ aryl, and the substituent may be halogen, cyano, hydroxyl, or C₆₋₁₄ aryl.

The term "therapeutically effective amount" refers to an amount of a compound that is sufficient to effectively treat the disease or condition described herein when administered to a subject. Although the amount of the compound constituting a "therapeutically effective amount" will vary depending on the compound, the condition and its severity, and the age of the subject to be treated, it can be determined by those skilled in the art in a conventional manner.

All terms subject, individual, and individual in need thereof refer to any animal, preferably a mammal, and most preferably a human, that is about to receive or has received administration of the conjugate or pharmaceutical composition according to an embodiment of the present disclosure. As used herein, the term "mammal" includes any mammal. Examples of mammals include, but are not limited to, cows, horses, sheep, pigs, cats, dogs, mice, rats, rabbits, guinea pigs, monkeys, humans, etc., with humans being most preferred.

In certain embodiments, "treatment" or "treating" refers to the amelioration, prevention, or reversal of a disease or condition or at least one discernible symptom thereof. In other embodiments, "treatment" or "treating" refers to the amelioration, prevention, or reversal of at least one measurable physical parameter of the disease or condition being treated, and the disease or condition may not be identified in the mammal. In yet another embodiment, "treatment" or "treating" refers to slowing the progression of the disease or condition, either physically (e.g., stabilization of discernible symptoms) or physiologically (e.g., stabilization of physical parameters), or both. In other embodiments, "treatment" or "treating" refers to delaying the onset of the disease or condition.

In certain embodiments, the compounds of the present disclosure may be administered as a preventive measure. As used herein, "prevention" or "preventing" refers to reducing the risk of acquiring a given disease or condition. In a preferred mode of the embodiments, the specified compound is administered as a preventive measure to a subject, such as a subject with a family history or predisposition to cancer or autoimmune diseases.

On the basis of not violating common knowledge in the art, the above preferred conditions can be arbitrarily combined to obtain preferred examples of the present disclosure.

The reagents and raw materials used in the present disclosure are all commercially available.

The GalNAc compounds provided in the present application and the GalNAc-conjugated oligonucleotides prepared therefrom exhibit one or more of the following beneficial effects:
1. The present application designs a series of novel GalNAc compounds, which are entirely different in chemical structure from GalNAc compounds in the prior art.
2. The GalNAc-conjugated oligonucleotides prepared from the GalNAc compounds designed in present application demonstrate significantly improved delivery efficiency compared to those prepared from GalNAc compounds (e.g., L96 and YK-GAL-325) in the prior art, with significantly improved inhibition rates of AGT protein expression in mouse serum and AGT mRNA levels in the liver.

### DETAILED DESCRIPTION OF THE INVENTION

In order to make the purpose, technical solutions, and advantages of the examples of the present disclosure clearer, the technical solutions of the examples of the present disclosure will be clearly and completely described below. Apparently, the described examples are some of the examples of the present disclosure, not all of them. Based on the described examples of the present disclosure, all other examples obtained by those of ordinary skill in the art without the need for creative effort shall fall within the scope of protection of the present disclosure.

The present disclosure may be embodied in other specific forms without departing from the essential attributes of the present disclosure. It should be understood that any and all embodiments of the present disclosure may be combined with technical features in any other embodiment or a plurality of other embodiments to obtain additional embodiments under the premise of no conflict. The present disclosure includes additional embodiments obtained from such combinations.

All publications and patents mentioned in the present disclosure are incorporated herein by reference in their entirety. If usage or terminology used in any publications and patents incorporated by reference conflicts with usage or terminology used in the present disclosure, the usage and terminology in the present disclosure shall prevail.

The section headings used herein are for the sole purpose of organizing the article and should not be construed as a limitation on the subject matter described.

Unless otherwise specified, all technical and scientific terms used herein have their ordinary meanings in the art to which the claimed subject matter pertains. If there are multiple definitions for a term, the definition herein shall prevail.

Except in the working examples or otherwise indicated, all numbers stating quantitative properties, such as doses, in the specification and claims should be understood as modified in all instances by the term "about". It should also be understood that any numerical range recited in the present disclosure is intended to include all sub-ranges within the range and any combination of the various endpoints of the range or sub-ranges.

The words "comprising", "including", or "containing" and similar words used in the present disclosure mean that the element appearing before the word covers the elements listed after the word and their equivalents, and does not exclude unrecited elements. The term "comprising" or "including (containing)" as used herein can be open, semi-closed, and closed. In other words, the term also includes "consisting essentially of" or "consisting of".

The present disclosure is further described below in conjunction with examples. However, the present disclosure is not limited to the following examples. The implementation conditions used in the examples can be further adjusted according to different requirements of specific applications, and the unspecified implementation conditions are conventional conditions in the industry. In the specific examples of the present disclosure, all raw materials used can be obtained commercially. Unless otherwise specified, all temperatures are given in degrees Celsius. The technical features in various embodiments of the present disclosure may be combined with each other as long as they do not conflict with one another.

### Preparation Example: Synthesis of GalNAc Compounds

The following abbreviations represent the following reagents: TEA: triethylamine; DCM: dichloromethane; 3A MS: 3A molecular sieves; MeONa: sodium methoxide; MeOH: methanol; Imidazole: imidazole; Pyridine: pyridine; NaOH: sodium hydroxide; DIPEA: diisopropylethylamine; THF: tetrahydrofuran; BF₃ Et₂O: boron trifluoride diethyl etherate; DMTrCl: 4,4'-dimethoxytrityl chloride; HBTU: *O*-(benzotriazol-1-yl)-*N*,*N*,*N'*,*N*'-tetramethyluronium hexafluorophosphate; DIPEA: N, N-diisopropylethylamine; DMAP: 4-dimethylaminopyridine; TIPDSCl: 1,3-dichloro-1,1,3,3-tetraisopropyldisiloxane; TEA 3HF: triethylamine trihydrofluoride; Proton Sponge: 1,8-bis(dimethylamino)naphthalene; Me₃OBF₄: trimethyloxonium tetrafluoroborate; DMF: N,N-dimethylformamide; Ac₂O: acetic anhydride.

### 1. Synthesis of GalNAc Phosphoramidite Compound

### (1) Synthesis of YK-GAL-501

### The synthetic route is as follows:

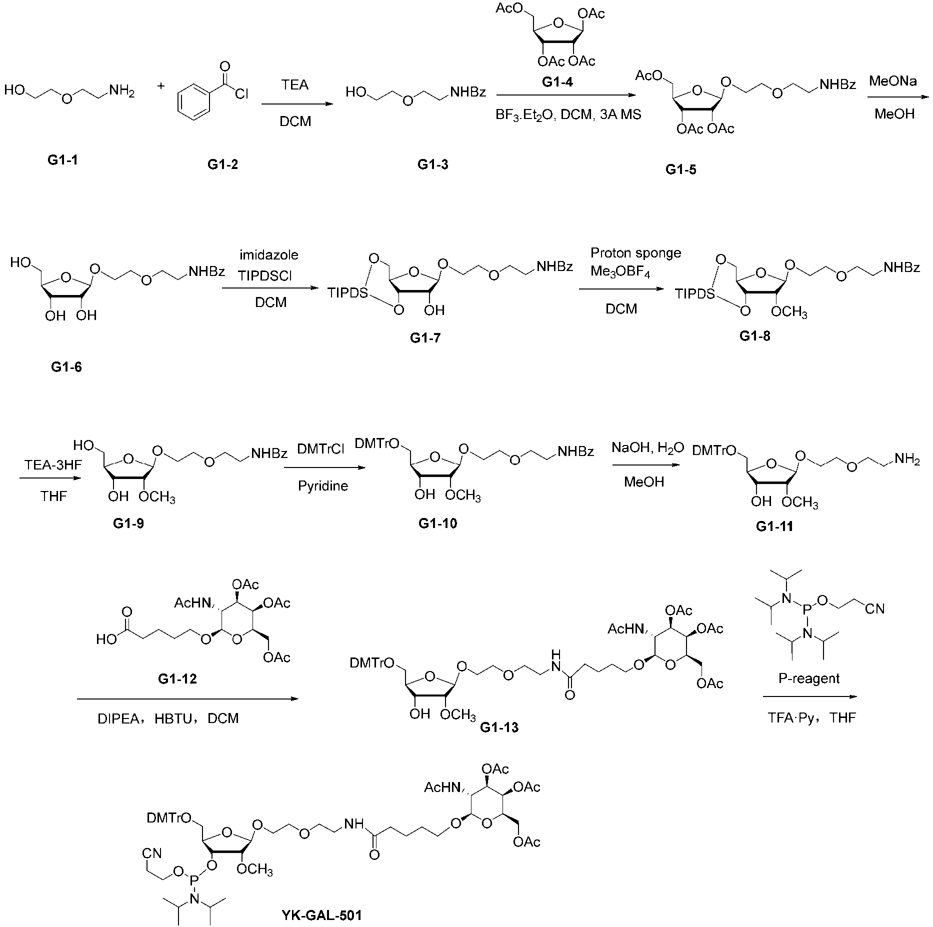

### Step 1: Synthesis of G1-3

Dichloromethane (1500 mL) was added to a dried compound G1-1 (100.0 g, 951.2 mmol), followed by the addition of triethylamine (107.3 g, 1.06 mol). The mixture was cooled to 0°C, and G1-2 (121.7 g, 865.1 mmol) was slowly added dropwise while maintaining the temperature at 0-5°C. After the dropwise addition, the mixture was warmed to room temperature and stirred for an additional 2 h. After being cooled to 0°C, 1 mol/L hydrochloric acid was added dropwise to adjust the pH to 6-7. A saturated aqueous sodium chloride solution (300 mL) was added, and the mixture was shaken and separated. The aqueous phase was extracted with dichloromethane (800 mL × 3), and the organic phases were combined. The organic phase was dried over anhydrous sodium sulfate and concentrated under reduced pressure to remove the solvent to obtain a crude product G1-3 (105.0 g) as a colorless oil, which was directly used in the next step. 1H NMR (400 MHz, DMSO-d6) δ 8.48 (t, J = 5.6 Hz, 1H), 7.84 (dd, J = 7.2, 1.9 Hz, 2H), 7.56 - 7.49 (m, 1H), 7.46 (dd, J = 8.2, 6.5 Hz, 2H), 4.42 (s, 1H), 3.57 - 3.48 (m, 4H), 3.48 - 3.38 (m, 4H). MS (ESI) m/z [M + H]⁺ =210.4.

### Step 2: Synthesis of G1-5

Dichloromethane (450 mL) was added to compound G1-4 (87.2 g, 274.0 mmol), and the mixture was stirred until a clear solution was obtained. 3A molecular sieves (80.0 g) were added, and the temperature was reduced to 0°C. Boron trifluoride diethyl etherate (116.9 g, 823.7 mmol) was then added. After the addition, the mixture was stirred for 20 min. A solution of G1-3 (86.0 g, 411.0 mmol) in dichloromethane (180 mL) was added dropwise to the system while maintaining the temperature at 0-5°C. After the dropwise addition, the mixture was stirred overnight at 0-5°C. The mixture was filtered, and a saturated aqueous sodium bicarbonate solution (125 mL) was added dropwise to the filtrate to quench the reaction while maintaining the temperature at 0-5°C. The phases were separated, and the organic phase was washed sequentially with saturated aqueous sodium bicarbonate solution (900 mL × 2) and half-saturated aqueous sodium chloride solution (900 mL). The organic phase was dried over anhydrous sodium sulfate and concentrated under reduced pressure to remove the solvent to obtain a crude product G1-5 (150.0 g) as a yellow oil, which was directly used in the next step. 1H NMR (400 MHz, DMSO-d6) δ 8.47 (t, J = 5.6 Hz, 1H), 7.84 (dt, J = 7.0, 1.4 Hz, 2H), 7.56 - 7.48 (m, 1H), 7.45 (dd, J = 8.2, 6.6 Hz, 2H), 5.17 (dd, J = 6.7, 4.9 Hz, 1H), 5.12 - 5.05 (m, 2H), 4.28 (dd, J = 11.7, 3.9 Hz, 1H), 4.25 - 4.18 (m, 1H), 4.04 (dd, J = 11.7, 5.6 Hz, 1H), 3.71 (qd, J = 8.6, 7.4, 4.4 Hz, 1H), 3.62 - 3.50 (m, 5H), 3.42 (q, J = 5.9 Hz, 2H), 2.06 (s, 3H), 2.02 (d, J = 2.0 Hz, 6H). MS (ESI) m/z [M + Na]⁺ =490.2.

### Step 3: Synthesis of G1-6

G1-5 (150.0 g, 320.9 mol) was dissolved in methanol (1200 mL), and the system was cooled to 0°C. A solution (300 mL) of sodium methoxide (3.50 g, 64.8 mmol) in methanol was added dropwise while maintaining the temperature at 0-5°C. After the dropwise addition, the mixture was warmed to room temperature and stirred for 1 h. The mixture was cooled to 0-5°C, and 1 mol/L hydrochloric acid was added dropwise to adjust the pH to 6-7 while maintaining the temperature at 0-5°C. The system was concentrated under reduced pressure to remove the solvent. Purified water (200 mL) was added, and the mixture was washed with dichloromethane (400 mL × 8). The aqueous phase was collected and concentrated under reduced pressure to remove the solvent to obtain a crude product. The crude product was purified by column chromatography (dichloromethane/methanol) to obtain G1-6 (69.0 g, 202.1 mmol) as a colorless transparent oil in a two-step combined yield of 73.8%. 1H NMR (400 MHz, DMSO-d6) δ 8.48 (t, J = 5.6 Hz, 1H), 7.88 - 7.81 (m, 2H), 7.56 - 7.49 (m, 1H), 7.46 (dd, J = 8.1, 6.5 Hz, 2H), 4.89 (d, J = 1.5 Hz, 1H), 4.83 (d, J = 6.9 Hz, 1H), 4.54 (t, J = 5.7 Hz, 1H), 4.30 (dd, J = 6.9, 4.4 Hz, 1H), 3.98 (td, J = 6.6, 4.7 Hz, 1H), 3.77 - 3.67 (m, 2H), 3.56 - 3.46 (m, 6H), 3.35 (s, 4H). MS (ESI) m/z [M - H]⁻ =340.3.

### Step 4: Synthesis of G1-7

G1-6 (69.0 g, 202.1 mmol) was dissolved in dichloromethane/tetrahydrofuran (5/1, 700 mL), followed by the addition of imidazole (34.4 g, 505.3 mmol). The mixture was cooled to 0°C, and 1,3-dichloro-1,1,3,3-tetraisopropyldisiloxane (66.8 g, 211.8 mmol) was added dropwise while maintaining the temperature at 0-5°C. After the dropwise addition, the mixture was stirred for 10 min, warmed to room temperature, and stirred for an additional 2 h. After the reaction was completed, purified water (700 mL) was slowly added dropwise to the reaction mixture. After the dropwise addition, the mixture was shaken and separated. The organic phase was washed with saturated aqueous sodium chloride solution (700 mL), dried over anhydrous sodium sulfate, and concentrated under reduced pressure to remove the solvent to obtain a crude product. n-Heptane (700 mL) was added for slurrying, followed by filtration. The filter cake was collected and dried under vacuum to obtain G1-7 (74.3 g, 127.3 mmol) as a white solid in a yield of 63.0%. 1H NMR (400 MHz, DMSO-d6) δ 8.46 (t, J = 5.6 Hz, 1H), 7.87 - 7.80 (m, 2H), 7.56 - 7.41 (m, 3H), 5.06 (d, J = 3.9 Hz, 1H), 4.76 (s, 1H), 4.30 (dd, J = 6.9, 4.4 Hz, 1H), 3.92 - 3.75 (m, 4H), 3.67 - 3.36 (m, 8H), 1.08 - 0.84 (m, 28H). MS (ESI) m/z [M - H]⁻ =582.5.

### Step 5: Synthesis of G1-8

G1-7 (70.0 g, 119.9 mmol) was dissolved in dichloromethane (700 mL), followed by the addition of 1,8-bis(dimethylamino)naphthalene (64.0 g, 298.6 mmol). The mixture was cooled to 0°C, and trimethyloxonium tetrafluoroborate (35.5 g, 240.0 mmol) was added. The mixture was stirred for 10 min, warmed to room temperature, and stirred for an additional 2 h. After the reaction was completed, purified water (5.4 mL) was added to quench the reaction. The mixture was stirred for 10 min, filtered, and the filtrate was collected and concentrated under reduced pressure to remove the solvent. *n*-Heptane (700 mL) was then added, and the mixture was stirred for 20 min, filtered again, and the filtrate was collected. The filtrate was washed once with 15wt% aqueous ammonium chloride solution (700 mL) and once with saturated aqueous sodium chloride solution (700 mL). The organic phase was dried over anhydrous sodium sulfate and concentrated under reduced pressure to remove the solvent to obtain a crude product. The crude product was purified by column chromatography (*n-*hexane/ethyl acetate) to obtain G1-8 (56.0 g, 93.7 mmol) as a wine-red oil in a yield of 78.1%. 1H NMR (400 MHz, DMSO-d6) δ 8.46 (t, J = 5.6 Hz, 1H), 7.87 - 7.80 (m, 1H), 7.56 - 7.28 (m, 4H), 4.86 (s, 1H), 4.40 (dd, J = 6.9, 4.2 Hz, 1H), 3.92 - 3.84 (m, 1H), 3.81 (dt, J = 7.2, 5.4 Hz, 2H), 3.68 - 3.34 (m, 11H), 2.99 - 2.90 (m, 1H), 1.01 (dt, J = 11.0, 5.6 Hz, 28H). MS (ESI) m/z [M - H]⁻ =596.5.

### Step 6: Synthesis of G1-9

G1-8 (56.0 g, 93.7 mmol) was dissolved in tetrahydrofuran (500 mL), cooled to 0°C, and triethylamine trihydrofluoride (45.3 g, 281.0 mmol) was slowly added dropwise. After the dropwise addition, the mixture was warmed to room temperature and stirred overnight at room temperature. After the reaction was completed, a saturated aqueous sodium bicarbonate solution (600 mL) was added, and the mixture was stirred for 5 min. A saturated aqueous sodium chloride solution (600 mL) was added, and the mixture was shaken and separated. The aqueous phase was extracted with tetrahydrofuran (600 mL). The combined organic phases were washed with a saturated aqueous sodium chloride solution (300 mL), then dried over anhydrous sodium sulfate, and concentrated under reduced pressure to remove the solvent to obtain a crude product. The crude product was dissolved in acetonitrile (300 mL) and washed with *n*-heptane (600 mL × 3). The acetonitrile phase was concentrated to obtain G1-9 (35.0 g) as a pale yellow oil, which was directly used in the next step. 1H NMR (400 MHz, DMSO-d6) δ 8.48 (t, J = 5.6 Hz, 1H), 7.88 - 7.81 (m, 2H), 7.56 - 7.49 (m, 1H), 7.46 (dd, J = 8.1, 6.5 Hz, 2H), 4.89 (d, J = 1.5 Hz, 1H), 4.83 (d, J = 6.9 Hz, 1H), 4.54 (t, J = 5.7 Hz, 1H), 3.98 (td, J = 6.6, 4.7 Hz, 1H), 3.77 - 3.67 (m, 2H), 3.56 - 3.46 (m, 6H), 3.46 - 3.39 (m, 3H), 3.35 (s, 4H). MS (ESI) m/z [M - H]⁻ =354.2.

### Step 7: Synthesis of G1-10

Dried G1-9 (35.0 g, 98.5 mmol) and 4-dimethylaminopyridine (1.20 g, 9.82 mmol) were dissolved in pyridine (350 mL). 4,4'-Dimethoxytrityl chloride (40.1 g, 118.3 mmol) was added in 4 batches, and the mixture was stirred at 15-20°C for 1 h. After the reaction was completed, the mixture was cooled to 0°C, and a saturated aqueous sodium bicarbonate solution (350 mL) was added dropwise to quench the reaction while maintaining the temperature at 0-5°C. After the dropwise addition, the mixture was stirred for 10 min. Dichloromethane (350 mL) was added, and the mixture was shaken and separated. The organic phase was washed once with purified water (350 mL) and then once with a 10wt% aqueous sodium chloride solution (350 mL). The organic phase was dried over anhydrous sodium sulfate and concentrated under reduced pressure to remove the solvent to obtain a crude product. The crude product was purified by column chromatography (*n*-heptane/ethyl acetate, 0.1% triethylamine) to obtain G1-10 (50.0 g, 76.0 mmol) as a pale yellow foamy solid in a yield of 77.2%. 1H NMR (400 MHz, DMSO-d6) δ 8.46 (t, J = 5.6 Hz, 1H), 7.87 - 7.80 (m, 2H), 7.56 - 7.48 (m, 1H), 7.48 - 7.39 (m, 4H), 7.33 - 7.24 (m, 6H), 7.24 - 7.15 (m, 1H), 6.91 - 6.84 (m, 4H), 4.98 (s, 1H), 4.87 (d, J = 7.3 Hz, 1H), 4.01 (td, J = 8.4, 7.3, 5.7 Hz, 1H), 3.91 (td, J = 7.0, 6.6, 2.8 Hz, 1H), 3.73 (s, 6H), 3.71 - 3.64 (m, 1H), 3.61 - 3.35 (m, 11H), 3.10 (dd, J = 10.0, 2.7 Hz, 1H), 2.96 (dd, J = 10.0, 6.0 Hz, 1H). MS (ESI) m/z [M - H]⁻ =656.6.

### Step 8: Synthesis of G1-11

G1-10 (50.0 g, 76.0 mmol) was dissolved in methanol (500 mL), followed by the addition of sodium hydroxide (136.8 g, 3.42 mol) and purified water (27.4 g, 1.52 mol). The mixture was sealed and heated to 130°C, and the reaction was carried out for 2 h. After the reaction was completed, the mixture was cooled to room temperature. Dichloromethane (1000 mL) and a 15wt% aqueous ammonium chloride solution (1000 mL) were added, and the mixture was stirred for 5 min. The phases were separated, and the organic phase was washed sequentially with purified water (1000 mL) and saturated aqueous sodium chloride solution (500 mL). The organic phase was dried over anhydrous sodium sulfate and then concentrated under reduced pressure to remove the solvent to obtain a crude product G1-11 (40.0 g) as a pale yellow solid, which was directly used in the next step. 1H NMR (400 MHz, DMSO-d6) δ 7.43 (d, J = 7.5 Hz, 2H), 7.29 (dd, J = 8.9, 2.8 Hz, 6H), 7.21 (t, J = 7.2 Hz, 1H), 6.88 (d, J = 8.5 Hz, 4H), 4.99 (s, 1H), 4.92 (s, 1H), 4.02 (dd, J = 7.4, 4.5 Hz, 1H), 3.93 (dt, J = 7.6, 3.7 Hz, 1H), 3.73 (s, 7H), 3.55 (ddd, J = 10.9, 6.9, 3.9 Hz, 1H), 3.44 (ddd, J = 15.2, 7.1, 4.3 Hz, 6H), 3.29 (t, J = 5.8 Hz, 2H), 3.11 (dd, J = 9.9, 2.8 Hz, 1H), 2.98 (dd, J = 10.0, 6.0 Hz, 1H), 2.59 (t, J = 5.8 Hz, 2H). MS (ESI) m/z [M + Na]⁺=576.4.

### Step 9: Synthesis of G1-13

G1-11 (40.0 g, 72.2 mmol) was dissolved in dichloromethane (400 mL), followed by the addition of *O*-(benzotriazol-1-yl)-*N*,*N*,*N*',*N*'-tetramethyluronium hexafluorophosphate (41.1 g, 108.4 mmol). The mixture was cooled to 0°C, and diisopropylethylamine (18.7 g, 144.7 mmol) was added. After stirring for 10 min, a solution of G1-12 (35.6 g, 79.6 mmol) in dichloromethane (400 mL) was slowly added dropwise. After the dropwise addition, the mixture was stirred for 1 h. After the reaction was completed, purified water (1000 mL) was added dropwise at 0-5°C. The mixture was shaken and separated, and the organic phase was washed with a 10wt% aqueous sodium chloride solution (1000 mL). The organic phase was dried over anhydrous sodium sulfate and then concentrated under reduced pressure to remove the solvent to obtain a crude product. The crude product was purified by column chromatography (dichloromethane/tetrahydrofuran, 0.1% triethylamine) to obtain G1-13 (28.9 g, 29.4 mmol) as a pale yellow foamy solid. Yield: 40.7%. 1H NMR (400 MHz, DMSO-d6) δ 7.83 - 7.72 (m, 2H), 7.42 (d, J = 7.2 Hz, 2H), 7.34 - 7.17 (m, 7H), 6.88 (d, J = 8.6 Hz, 4H), 5.21 (d, J = 3.4 Hz, 1H), 4.98 (s, 2H), 4.88 (d, J = 7.3 Hz, 1H), 4.48 (d, J = 8.5 Hz, 1H), 4.02 (q, J = 4.2 Hz, 4H), 3.89 (ddt, J = 19.9, 11.0, 5.6 Hz, 2H), 3.74 (s, 6H), 3.68 (dq, J = 9.5, 4.5 Hz, 2H), 3.58 - 3.32 (m, 10H), 3.19 - 3.06 (m, 3H), 2.95 (dd, J = 10.0, 6.0 Hz, 1H), 2.10 (s, 3H), 2.05 (t, J = 7.1 Hz, 2H), 1.99 (s, 3H), 1.89 (s, 3H), 1.77 (s, 3H), 1.47 (d, J = 14.5 Hz, 4H). MS (ESI) m/z [M - H]⁻=981.8.

### Step 10: Synthesis of YK-GAL-501

G1-13 (3.0 g, 3.05 mmol) was dissolved in tetrahydrofuran (15 mL), followed by the addition of 3A molecular sieves (750 mg). The mixture was stirred and dried for 30 min, and designated as solution A for later use; Pyridinium trifluoroacetate (1.18 g, 6.11 mmol) and triphenylphosphine (80 mg, 0.31 mmol) were dissolved in tetrahydrofuran (15 mL), followed by the addition of 3A molecular sieves (750 mg). The mixture was stirred and dried for 30 min, and designated as solution B for later use. Solution B was cooled to 0°C, and a phosphorus reagent, bis(diisopropylamino)(2-cyanoethoxy)phosphine (2.30 g, 7.63 mmol), was added. Then, solution A was added dropwise to solution B, and the mixture was stirred for 1 h. After the reaction was completed, the mixture was filtered. 5wt% Aqueous sodium bicarbonate solution (30 mL) was added to the filtrate, and the mixture was stirred for 5 min. Dichloromethane (60 mL) was added, and the mixture was shaken and separated. The organic phase was washed sequentially with 5wt% aqueous sodium bicarbonate solution (30 mL) and saturated aqueous sodium chloride solution (30 mL). The organic phase was dried over anhydrous sodium sulfate and concentrated to remove the solvent to obtain a residue. The residue was dissolved in dichloromethane (3 mL) and added dropwise to n-heptane (60 mL) at 10-15°C. The mixture was stirred for 30 min, and the supernatant was removed to obtain a viscous oily solid, which was dried under vacuum to obtain YK-GAL-501 (3.4 g, 2.87 mmol) as a white foamy solid in a yield of 94.4%. 1H NMR (400 MHz, DMSO-d6) δ 7.79 (d, J = 9.2 Hz, 1H), 7.74 (t, J = 5.6 Hz, 1H), 7.47 - 7.37 (m, 2H), 7.34 - 7.17 (m, 7H), 6.91 - 6.83 (m, 4H), 5.21 (d, J = 3.4 Hz, 1H), 5.04 (d, J = 5.1 Hz, 1H), 4.97 (dd, J = 11.2, 3.4 Hz, 1H), 4.49 (d, J = 8.5 Hz, 1H), 4.13 - 3.96 (m, 5H), 3.92 - 3.82 (m, 1H), 3.73 (d, J = 2.0 Hz, 8H), 3.61 (dd, J = 14.9, 4.6 Hz, 2H), 3.53 - 3.33 (m, 11H), 3.21 - 3.09 (m, 2H), 2.95 (dd, J = 10.5, 5.2 Hz, 1H), 2.89 (t, J = 5.8 Hz, 2H), 2.75 (t, J = 6.1 Hz, 1H), 2.56 (t, J = 5.9 Hz, 1H), 2.10 (s, 3H), 2.03 (q, J = 6.4 Hz, 2H), 1.99 (s, 3H), 1.89 (s, 3H), 1.77 (s, 3H), 1.45 (s, 4H), 1.26 - 1.04 (m, 12H).MS (ESI) m/z [M + H]⁻ = 1184.2.

### (2) Synthesis of YK-GAL-502

The synthetic route is as follows:

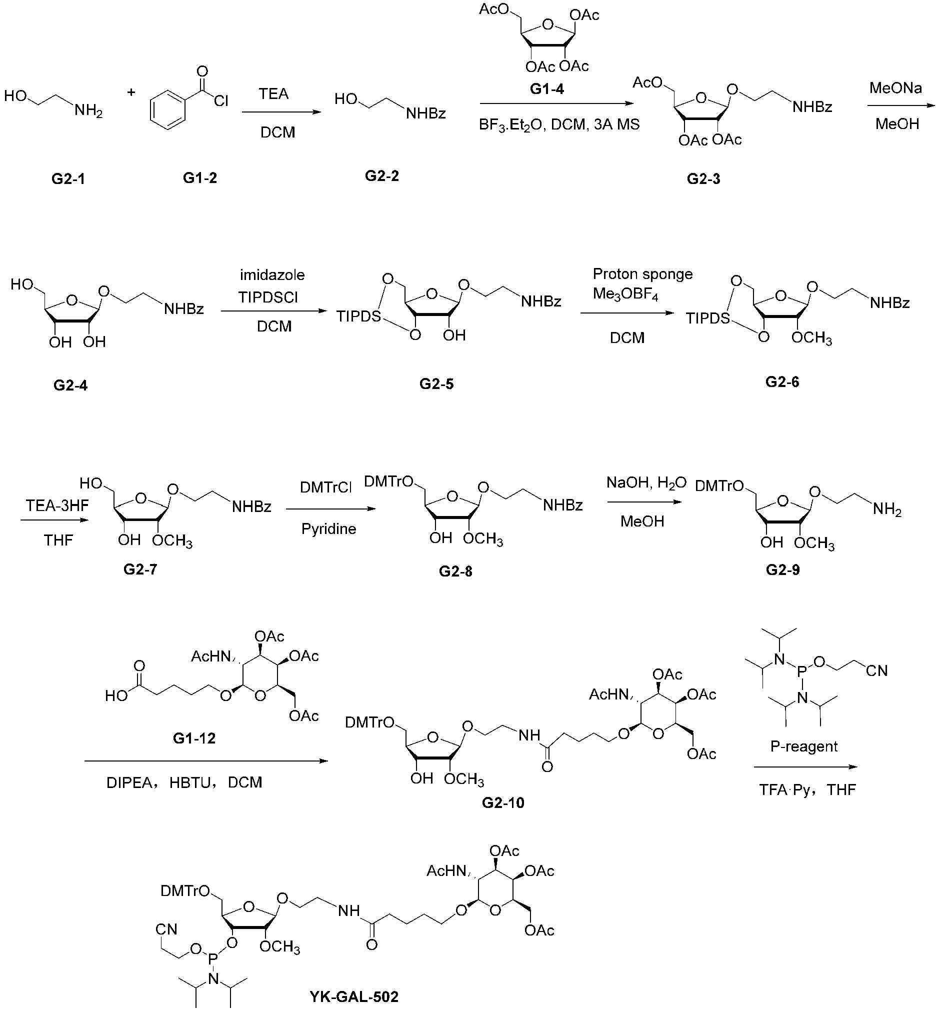

### Step 1: Synthesis of G2-2

Using **G2-1** (30.4 g, 497.8 mmol) and **G1-2** (70.0 g, 498.0 mmol) as starting materials and following the synthesis method for **G1-3,** a crude product **G2-2** (70.0 g) was obtained and directly used in the next step. 1H NMR (400 MHz, DMSO-d6) δ 8.45 (t, J = 5.5 Hz, 1H), 7.90 - 7.83 (m, 2H), 7.56 - 7.41 (m, 3H), 4.75 (t, J = 5.6 Hz, 1H), 3.52 (q, J = 6.0 Hz, 2H), 3.35 (t, J = 6.0 Hz, 2H). MS (ESI) m/z [M + H]⁺ =166.3.

### Step 2: Synthesis of G2-3

Using **G1-4** (77.0 g, 241.9 mmol) and **G2-2** (60.0 g, 363.2 mmol) as starting materials and following the synthetic method for **G1-5,** a crude product **G2-3** (100.0 g) was obtained and directly used in the next step. 1H NMR (400 MHz, Chloroform-d) δ 7.84 - 7.77 (m, 2H), 7.54 - 7.47 (m, 1H), 7.43 (dd, J = 8.2, 6.6 Hz, 2H), 6.78 (s, 1H), 5.31 (dd, J = 6.3, 4.9 Hz, 1H), 5.25 (dd, J = 4.9, 1.4 Hz, 1H), 5.05 (d, J = 1.4 Hz, 1H), 4.32 (dt, J = 9.5, 3.6 Hz, 2H), 3.86 (ddd, J = 10.2, 6.4, 3.5 Hz, 1H), 3.79 - 3.57 (m, 3H), 2.08 (d, J = 14.2 Hz, 6H), 2.02 (s, 3H). MS (ESI) m/z [M + Na]⁺ =446.2

### Step 3: Synthesis of G2-4

Using **G2-3** (100.0 g, 236.2 mol) as the starting material and following the synthetic method for **G1-6, G2-4** (40.0 g, 135.4 mmol) was obtained as a colorless transparent oil in a two-step combined yield of 56.0%. 1H NMR (400 MHz, DMSO-d6) δ 8.43 (t, J = 5.6 Hz, 1H), 7.86 - 7.80 (m, 2H), 7.52 (t, J = 7.3 Hz, 1H), 7.45 (dd, J = 8.3, 6.6 Hz, 2H), 4.99 (d, J = 4.5 Hz, 1H), 4.78 (d, J = 6.9 Hz, 2H), 4.65 (t, J = 5.6 Hz, 1H), 3.89 (td, J = 6.7, 4.7 Hz, 1H), 3.75 (qd, J = 8.1, 7.1, 3.4 Hz, 2H), 3.68 (dd, J = 11.0, 5.0 Hz, 1H), 3.52 (tt, J = 10.0, 4.6 Hz, 2H), 3.45 - 3.33 (m, 3H). MS (ESI) m/z [M - H]⁻=296.3.

### Step 4: Synthesis of G2-5

Using **G2-4** (34.0 g, 115.1 mmol) as the starting material and following the synthetic method for **G1-7, G2-5** (50.0 g, 92.6 mmol) was obtained as a white solid in a yield of 80.5%. 1H NMR (400 MHz, DMSO-d6) δ 8.41 (t, J = 5.6 Hz, 1H), 7.86 - 7.79 (m, 2H), 7.52 (t, J = 7.3 Hz, 1H), 7.44 (t, J = 7.5 Hz, 2H), 5.08 (d, J = 4.0 Hz, 1H), 4.78 (s, 1H), 4.30 (dd, J = 7.7, 4.3 Hz, 1H), 3.91 - 3.74 (m, 4H), 3.68 - 3.59 (m, 1H), 3.49 (dt, J = 9.9, 6.0 Hz, 1H), 3.39 (dt, J = 8.7, 5.9 Hz, 2H), 1.06 - 0.86 (m, 28H). MS (ESI) m/z [M - H]⁻=538.4.

### Step 5: Synthesis of G2-6

Using **G2-5** (43.0 g, 79.7 mmol) as the starting material and following the synthetic method for **G1-8, G2-6** (31.0 g, 56.0 mmol) was obtained as a wine-red oil in a yield of 70.3%. 1H NMR (400 MHz, DMSO-d6) δ 8.40 (t, J = 5.6 Hz, 1H), 7.87 - 7.80 (m, 2H), 7.52 (t, J = 7.2 Hz, 1H), 7.45 (t, J = 7.5 Hz, 2H), 4.90 (s, 1H), 4.41 (dd, J = 7.6, 4.1 Hz, 1H), 3.90 - 3.75 (m, 3H), 3.71 - 3.58 (m, 2H), 3.53 (dt, J = 10.0, 5.9 Hz, 1H), 3.45 (s, 3H), 3.41 (t, J = 5.9 Hz, 2H), 1.01 (ddt, J = 13.9, 7.1, 3.2 Hz, 28H). MS (ESI) m/z [M - H]⁻=552.4.

### Step 6: Synthesis of G2-7

Using **G2-6** (31.0 g, 56.0 mmol) as the starting material and following the synthetic method for **G1-9, G2-7** (17.5 g) was obtained as a pale yellow oil, which was directly used in the next step. 1H NMR (400 MHz, Chloroform-d) δ 7.83 - 7.76 (m, 2H), 7.54 - 7.46 (m, 1H), 7.43 (dd, J = 8.2, 6.6 Hz, 2H), 5.04 (d, J = 1.4 Hz, 1H), 4.34 (t, J = 5.5 Hz, 1H), 4.00 (dt, J = 6.2, 3.3 Hz, 1H), 3.82 (ddt, J = 17.2, 10.6, 3.2 Hz, 3H), 3.77 - 3.58 (m, 4H), 3.51 (s,3H). MS (ESI) m/z [M - H]⁻=310.2.

### Step 7: Synthesis of G2-8

Using dried **G2-7** (16.8 g, 54.0 mmol) as the starting material and following the synthetic method for **G1-10, G2-8** (19.0 g, 31.0 mmol) was obtained as a pale yellow foamy solid in a yield of 57.4%. 1H NMR (400 MHz, DMSO-d6) δ 8.42 (t, J = 5.6 Hz, 1H), 7.84 - 7.78 (m, 2H), 7.56 - 7.48 (m, 1H), 7.48 - 7.39 (m, 4H), 7.34 - 7.24 (m, 6H), 7.19 (t, J = 7.3 Hz, 1H), 6.87 (d, J = 8.4 Hz, 4H), 5.01 (s, 1H), 4.88 (d, J = 7.2 Hz, 1H), 4.01 (td, J = 7.2, 4.4 Hz, 1H), 3.92 (td, J = 7.4, 6.7, 2.7 Hz, 1H), 3.72 (d, J = 2.6 Hz, 7H), 3.66 - 3.58 (m, 1H), 3.47 (d, J = 4.6 Hz, 1H), 3.46 - 3.33 (m, 5H), 3.10 (dd, J = 10.1, 2.7 Hz, 1H), 2.98 (dd, J = 10.0, 6.0 Hz, 1H). MS (ESI) m/z [M - H]⁻=612.5.

### Step 8: Synthesis of G2-9

Using **G2-8** (18.0 g, 29.3 mmol) as the starting material and following the synthetic method for **G1-11,** a crude product **G2-9** (15.0 g) was obtained as a pale yellow solid, which directly used in the next step. 1H NMR (400 MHz, DMSO-d6) δ 7.46 - 7.39 (m, 2H), 7.34 - 7.25 (m, 6H), 7.24 - 7.18 (m, 1H), 6.92 - 6.83 (m, 4H), 4.94 (d, J = 1.2 Hz, 1H), 4.87 (d, J = 6.9 Hz, 1H), 4.00 (s, 1H), 3.91 (td, J = 6.7, 2.7 Hz, 1H), 3.73 (s, 6H), 3.58 (dt, J = 9.7, 5.6 Hz, 1H), 3.51 - 3.45 (m, 1H), 3.35 (dd, J = 9.6, 6.2 Hz, 4H), 3.09 (dd, J = 10.0, 2.8 Hz, 1H), 2.96 (dd, J = 10.0, 5.9 Hz, 1H), 2.59 (t, J = 5.9 Hz, 2H). MS (ESI) m/z [M +Na]⁺=532.4.

### Step 9: Synthesis of G2-10

Using **G2-9** (13.5 g, 26.5 mmol) and **G1-12** (13.0 g, 29.1 mmol) as starting materials and following the synthetic method for **G1-13, G2-10** (18.6 g, 19.8 mmol) was obtained as a pale yellow foamy solid in a yield of 74.7%. 1H NMR (400 MHz, DMSO-d6) δ 7.80 (d, J = 9.3 Hz, 1H), 7.71 (t, J = 5.6 Hz, 1H), 7.42 (d, J = 7.3 Hz, 2H), 7.34 - 7.25 (m, 6H), 7.21 (t, J = 7.3 Hz, 1H), 6.88 (d, J = 8.6 Hz, 4H), 5.21 (d, J = 3.4 Hz, 1H), 5.01 - 4.94 (m, 2H), 4.88 (d, J = 7.3 Hz, 1H), 4.48 (d, J = 8.5 Hz, 1H), 4.02 (d, J = 5.5 Hz, 4H), 3.95 - 3.83 (m, 2H), 3.74 (s, 6H), 3.71 - 3.64 (m, 1H), 3.64 - 3.54 (m, 1H), 3.46 (d, J = 4.6 Hz, 1H), 3.45 - 3.35 (m, 5H), 3.21 (dd, J = 13.5, 5.8 Hz, 1H), 3.16 - 3.06 (m, 2H), 2.96 (dd, J = 10.0, 6.0 Hz, 1H), 2.10 (s, 3H), 2.02 (d, J = 6.8 Hz, 2H), 1.99 (s, 3H), 1.89 (s, 3H), 1.77 (s, 3H), 1.46 (dt, J = 13.0, 7.4 Hz, 4H). MS (ESI) m/z [M - H] ⁻=937.7.

### Step 10: Synthesis of YK-GAL-502

Using **G2-10** (2.0 g, 2.13 mmol) as the starting material and following the synthetic method for **YK-GAL-501, YK-GAL-502** (2.0 g, 1.76 mmol) was obtained as a white foamy solid in a yield of 82.3%. 1H NMR (400 MHz, DMSO-d6) δ 7.79 (d, J = 9.2 Hz, 1H), 7.73 - 7.63 (m, 1H), 7.46 - 7.39 (m, 2H), 7.28 (t, J = 8.7 Hz, 6H), 7.22 (t, J = 5.5 Hz, 1H), 6.87 (dd, J = 8.7, 4.8 Hz, 4H), 5.21 (d, J = 3.4 Hz, 1H), 5.02 (d, J = 4.2 Hz, 1H), 4.97 (dd, J = 11.3, 3.5 Hz, 1H), 4.48 (d, J = 8.5 Hz, 1H), 4.02 (s, 5H), 3.93 - 3.81 (m, 1H), 3.73 (d, J = 1.9 Hz, 6H), 3.71 - 3.65 (m, 1H), 3.65 - 3.56 (m, 2H), 3.52 - 3.42 (m, 4H), 3.39 (d, J = 13.9 Hz, 4H), 3.17 (d, J = 40.4 Hz, 3H), 2.95 (s, 1H), 2.89 (t, J = 5.9 Hz, 1H), 2.75 (t, J = 5.9 Hz, 1H), 2.56 (t, J = 5.9 Hz, 1H), 2.10 (s, 3H), 2.02 (d, J = 6.8 Hz, 2H), 1.99 (s, 3H), 1.89 (s, 3H), 1.76 (s, 3H), 1.52 - 1.38 (m, 4H), 1.22 - 1.03 (m, 12H). MS (ESI) m/z [M + H]⁺=1140.1.

### 1) Synthesis of YK-GAL-503

### (3) Synthesis of YK-GAL-503

### The synthetic route is as follows:

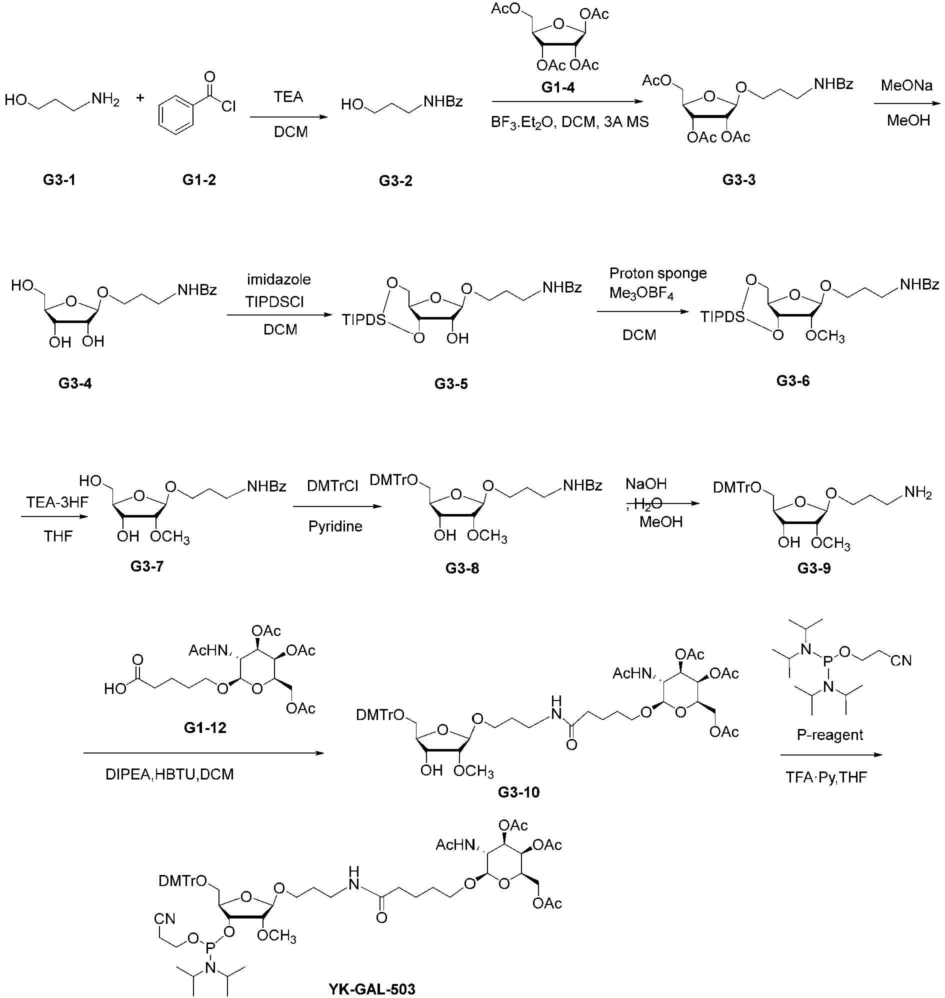

### Step 1: Synthesis of G3-2

Using G3-1 (22.5 g, 300.0 mmol) and G1-2 (42.2 g, 300.0 mmol) as starting materials and following the synthetic method for G1-3, a crude product G3-2 (63.0 g) was obtained as a colorless oil, which was directly used in the next step. 1H NMR (400 MHz, DMSO-d6) δ 8.42 (s, 1H), 7.87 - 7.79 (m, 2H), 7.55 - 7.48 (m, 1H), 7.45 (dd, J = 8.2, 6.4 Hz, 2H), 4.46 (t, J = 5.2 Hz, 1H), 3.31 (td, J = 6.9, 5.7 Hz, 4H), 1.67 (p, J = 6.5 Hz, 2H). MS (ESI) m/z [M + H]⁺ = 180.3.

### Step 2: Synthesis of G3-3

Using G1-4 (70.0 g, 219.9 mmol) and G3-2 (51.2 g, 285.9 mmol) as starting materials and following the synthetic method for G1-5, a crude product G3-3 (135.0 g) was obtained as a yellow oil, which was directly used in the next step. 1H NMR (400 MHz, DMSO-d6) δ 8.40 (t, J = 5.7 Hz, 1H), 7.89 - 7.80 (m, 2H), 7.49 - 7.45 (m, 1H), 7.43 (dd, J = 8.1, 6.4 Hz, 2H), 5.15 (dd, J = 6.6, 4.9 Hz, 1H), 5.10 (dd, J = 4.9, 1.4 Hz, 1H), 5.04 (d, J = 1.3 Hz, 1H), 4.30 - 4.21 (m, 2H), 4.04 (ddt, J = 10.9, 7.2, 3.2 Hz, 1H), 3.66 - 3.58 (m, 1H), 3.44 - 3.38 (m, 1H), 3.28 (t, J = 6.1 Hz, 2H), 2.06 (s, 3H), 2.08 (d, J = 3.6 Hz, 6H), 1.62 (p, J = 3.1 Hz, 2H). MS (ESI) m/z [M + Na]⁺ =460.2.

### Step 3: Synthesis of G3-4

Using G3-3 (135 g, 308.6 mol) as the starting material and following the synthetic method for G1-6, G3-4 (31.9 g, 102.5 mmol) was obtained as a colorless transparent oil in a two-step combined yield of 33.2%. 1H NMR (400 MHz, DMSO-d6) δ 8.42 (t, J = 5.7 Hz, 1H), 7.86 - 7.79 (m, 2H), 7.56 - 7.48 (m, 1H), 7.45 (dd, J = 8.2, 6.5 Hz, 2H), 4.97 (d, J = 4.5 Hz, 1H), 4.78 (d, J = 6.7 Hz, 1H), 4.74 (d, J = 1.2 Hz, 1H), 4.65 (t, J = 5.7 Hz, 1H), 3.87 (td, J = 6.7, 4.8 Hz, 1H), 3.80 - 3.71 (m, 2H), 3.67 (dt, J = 9.7, 6.4 Hz, 1H), 3.53 (ddd, J = 11.5, 5.7, 3.8 Hz, 1H), 3.41 - 3.34 (m, 2H), 3.29 (td, J = 6.9, 6.4, 2.4 Hz, 2H), 1.74 (p, J = 6.6 Hz, 2H). MS (ESI) m/z [M - H]⁻ =310.3.

### Step 4: Synthesis of G3-5

Using G3-4 (30.0 g, 96.4 mmol) as the starting material and following the synthetic method for G1-7, G3-5 (43.0 g, 77.6 mmol) was obtained as a white solid in a yield of 80.6%. 1H NMR (400 MHz, DMSO-d6) δ 8.38 (t, J = 5.7 Hz, 1H), 7.85 - 7.78 (m, 2H), 7.53 - 7.40 (m, 3H), 5.06 (d, J = 4.0 Hz, 1H), 4.74 (s, 1H), 4.30 (dd, J = 7.1, 4.4 Hz, 1H), 3.92 - 3.74 (m, 4H), 3.64 - 3.57 (m, 1H), 3.41 - 3.34 (m, 1H), 3.26 (dd, J = 10.0, 4.3 Hz, 2H), 1.77 - 1.68 (m, 2H), 1.07 - 0.85 (m, 28H). MS (ESI) m/z [M - H]⁻ =552.6.

### Step 5: Synthesis of G3-6

Using G3-5 (40.0 g, 72.2 mmol) as the starting material and following the synthetic method for G1-8, G3-6 (31.2 g, 54.9 mmol) was obtained as a wine-red oil in a yield of 76.0%. 1H NMR (400 MHz, DMSO-d6) δ 8.39 (t, J = 5.6 Hz, 1H), 7.86 - 7.79 (m, 2H), 7.51 (t, J = 7.3 Hz, 1H), 7.45 (dd, J = 8.2, 6.5 Hz, 2H), 4.85 (s, 1H), 4.42 (dd, J = 7.1, 4.3 Hz, 1H), 3.91 - 3.78 (m, 3H), 3.63 (dd, J = 9.7, 6.4 Hz, 1H), 3.59 (d, J = 4.3 Hz, 1H), 3.46 (s, 3H), 3.40 (dt, J = 9.7, 6.4 Hz, 1H), 3.33 - 3.25 (m, 2H), 1.74 (pd, J = 6.8, 2.0 Hz, 2H), 1.07 - 0.95 (m, 28H). MS (ESI) m/z [M - H]⁻ =566.6.

### Step 6: Synthesis of G3-7

Using G3-6 (30.0 g, 52.8 mmol) as the starting material and following the synthetic method for G1-9, G3-7 (19.1 g) was obtained as a pale yellow oil, which was directly used in the next step. 1H NMR (400 MHz, DMSO-d6) δ 8.42 (t, J = 5.6 Hz, 1H), 7.87 - 7.79 (m, 2H), 7.56 - 7.48 (m, 1H), 7.48 - 7.41 (m, 2H), 4.87 (d, J = 1.7 Hz, 1H), 4.82 (d, J = 6.9 Hz, 1H), 4.67 (t, J = 5.7 Hz, 1H), 3.98 (td, J = 6.5, 4.6 Hz, 1H), 3.78 - 3.63 (m, 2H), 3.56 - 3.47 (m, 1H), 3.45 (dd, J = 4.8, 1.7 Hz, 1H), 3.44 - 3.39 (m, 1H), 3.36 (s, 4H), 3.34 - 3.25 (m, 2H), 1.75 (p, J = 6.7 Hz, 2H). MS (ESI) m/z [M - H]⁻ =324.4.

### Step 7: Synthesis of G3-8

Using G3-7 (18.0 g, 55.3 mmol) as the starting material and following the synthetic method for G1-10, G3-8 (24.5 g, 39.0 mmol) was obtained as a pale yellow foamy solid in a yield of 70.6%. 1H NMR (400 MHz, DMSO-d6) δ 8.39 (t, J = 5.8 Hz, 1H), 7.81 (d, J = 7.6 Hz, 2H), 7.51 (dd, J = 8.3, 6.2 Hz, 1H), 7.44 (t, J = 8.2 Hz, 4H), 7.33 - 7.24 (m, 6H), 7.18 (t, J = 7.3 Hz, 1H), 6.91 - 6.84 (m, 4H), 4.96 (s, 1H), 4.87 (d, J = 7.1 Hz, 1H), 4.08 - 3.98 (m, 1H), 3.91 (t, J = 7.5 Hz, 1H), 3.71 (d, J = 1.9 Hz, 7H), 3.47 (d, J = 4.4 Hz, 2H), 3.39 (d, J = 1.7 Hz, 3H), 3.25 (p, J = 6.7 Hz, 2H), 3.10 (dd, J = 10.1, 2.7 Hz, 1H), 2.95 (dd, J = 10.1, 5.8 Hz, 1H), 1.71 (p, J = 6.8 Hz, 2H). MS (ESI) m/z [M - H]⁻ =626.4.

### Step 8: Synthesis of G3-9

Using G3-8 (23.0 g, 36.6 mmol) as the starting material and following the synthetic method for G1-11, a crude product G3-9 (18.5 g) was obtained as a pale yellow solid, which directly used in the next step. 1H NMR (400 MHz, DMSO-d6) δ 7.47 - 7.40 (m, 2H), 7.29 (dd, J = 8.7, 3.0 Hz, 6H), 7.25 - 7.17 (m, 1H), 6.92 - 6.84 (m, 4H), 4.92 (d, J = 1.2 Hz, 1H), 4.90 - 4.78 (m, 1H), 3.99 (dd, J = 7.2, 4.5 Hz, 1H), 3.91 (td, J = 7.6, 6.7, 2.7 Hz, 1H), 3.73 (s, 6H), 3.67 (dt, J = 9.5, 6.5 Hz, 1H), 3.48 - 3.35 (m, 5H), 3.09 (dd, J = 9.9, 2.8 Hz, 1H), 2.95 (dd, J = 9.9, 5.9 Hz, 1H), 2.53 - 2.43 (m, 2H), 1.50 (p, J = 6.7 Hz, 2H). MS (ESI) m/z [M + Na]⁺=546.4.

### Step 9: Synthesis of G3-10

Using G3-9 (18.0 g, 34.4 mmol) and G1-12 (16.9 g, 37.8 mmol) as starting materials and following the synthetic method for G1-13, G3-10 (23.0 g, 24.1 mmol) was obtained as a pale yellow foamy solid in a yield of 70.1%. 1H NMR (400 MHz, DMSO-d6) δ 7.80 (d, J = 9.2 Hz, 1H), 7.67 (t, J = 5.6 Hz, 1H), 7.46 - 7.39 (m, 2H), 7.34 - 7.24 (m, 6H), 7.21 (t, J = 7.3 Hz, 1H), 6.88 (d, J = 8.5 Hz, 4H), 5.22 (d, J = 3.4 Hz, 1H), 4.97 (dd, J = 11.3, 3.4 Hz, 1H), 4.93 (s, 1H), 4.86 (d, J = 7.2 Hz, 1H), 4.49 (d, J = 8.5 Hz, 1H), 4.02 (d, J = 5.8 Hz, 4H), 3.95 - 3.77 (m, 2H), 3.73 (s, 6H), 3.72 - 3.56 (m, 2H), 3.46 (d, J = 4.5 Hz, 1H), 3.41 (s, 5H), 3.10 (dd, J = 9.9, 2.7 Hz, 1H), 3.01 (p, J = 6.6 Hz, 2H), 2.94 (dd, J = 10.1, 5.8 Hz, 1H), 2.10 (s, 3H), 2.00 (d, J = 8.7 Hz, 5H), 1.89 (s, 3H), 1.76 (d, J = 3.8 Hz, 3H), 1.56 (p, J = 6.8 Hz, 2H), 1.47 (p, J = 7.1 Hz, 4H). MS (ESI) m/z [M - H]⁻=951.8.

### Step 10: Synthesis of YK-GAL-503

Using G3-10 (5.0 g, 5.25 mmol) as the starting material and following the synthetic method for YK-GAL-501, YK-GAL-503 (5.1 g, 4.42 mmol) was obtained as a white foamy solid in a yield of 84.3%. 1H NMR (400 MHz, DMSO-d6) δ 7.80 (d, J = 9.2 Hz, 1H), 7.66 (d, J = 6.1 Hz, 1H), 7.43 (dd, J = 7.7, 5.1 Hz, 2H), 7.29 (td, J = 8.9, 8.1, 3.6 Hz, 6H), 7.21 (dd, J = 8.4, 5.9 Hz, 1H), 6.87 (dd, J = 8.7, 4.2 Hz, 4H), 5.21 (d, J = 3.4 Hz, 1H), 4.97 (dd, J = 11.6, 3.6 Hz, 2H), 4.49 (d, J = 8.4 Hz, 1H), 4.13 - 3.95 (m, 5H), 3.93 - 3.80 (m, 1H), 3.73 (d, J = 2.0 Hz, 9H), 3.61 (dd, J = 16.2, 4.5 Hz, 1H), 3.56 - 3.35 (m, 7H), 3.21 (dd, J = 26.4, 10.1 Hz, 1H), 3.02 (d, J = 7.3 Hz, 2H), 2.89 (t, J = 5.9 Hz, 2H), 2.75 (t, J = 5.9 Hz, 1H), 2.56 (t, J = 5.9 Hz, 1H), 2.10 (s, 3H), 2.00 (d, J = 8.2 Hz, 5H), 1.89 (s, 3H), 1.77 (s, 3H), 1.58 (q, J = 6.3 Hz, 2H), 1.46 (d, J = 12.8 Hz, 4H), 1.21 - 1.02 (m, 12H). MS (ESI) m/z [M + H]⁺=1154.3.

### (4) Synthesis of YK-GAL-504

### The synthetic route is as follows:

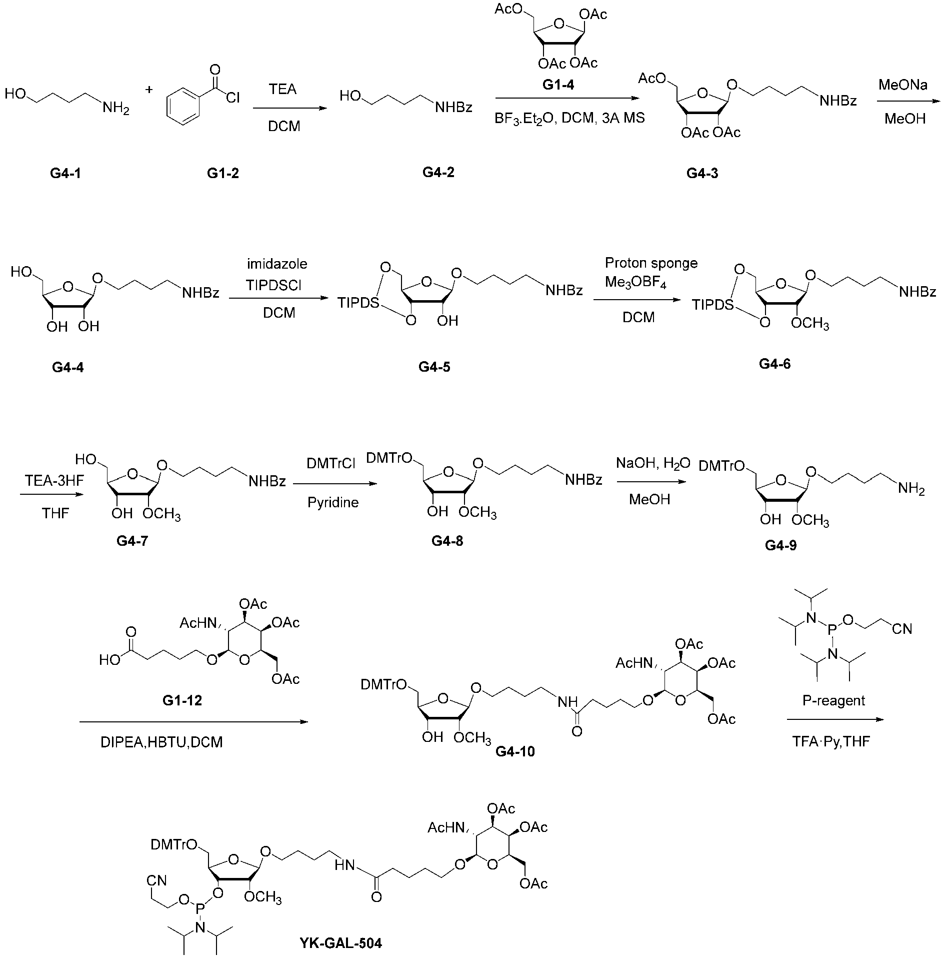

### Step 1: Synthesis of G4-2

Using G4-1 (60.0 g, 673.1 mmol) and G1-2 (94.6 g, 673.0 mmol) as starting materials and following the synthetic method for G1-3, a crude product G4-2 (135.0 g) was obtained as a colorless oil, which was directly used in the next step. 1H NMR (400 MHz, Chloroform-d) δ 7.77 (dt, J = 7.0, 1.4 Hz, 2H), 7.54 - 7.46 (m, 1H), 7.43 (dd, J = 8.2, 6.5 Hz, 2H), 6.48 (s, 1H), 3.73 (t, J = 5.9 Hz, 2H), 3.51 (t, J = 6.6 Hz, 2H), 1.78 - 1.65 (m, 4H). MS (ESI) m/z [M - H]⁻ =194.4.

### Step 2: Synthesis of G4-3

Using G1-4 (120.0 g, 377.0 mmol) and G4-2 (110 g, 569.2 mmol) as starting materials and following the synthetic method for G1-5, a crude product G4-3 (194.0 g) was obtained as a yellow oil, which was directly used in the next step. 1H NMR (400 MHz, DMSO-d6) δ 8.43 (t, J = 5.7 Hz, 1H), 7.87 - 7.80 (m, 2H), 7.55 - 7.48 (m, 1H), 7.45 (dd, J = 8.2, 6.5 Hz, 2H), 5.17 (dd, J = 6.5, 4.9 Hz, 1H), 5.09 (dd, J = 4.9, 1.2 Hz, 1H), 5.03 (d, J = 1.3 Hz, 1H), 4.32 - 4.19 (m, 2H), 4.04 (ddt, J = 10.8, 7.1, 3.2 Hz, 1H), 3.69 - 3.59 (m, 1H), 3.46 - 3.39 (m, 1H), 3.28 (t, J = 6.0 Hz, 2H), 2.07 (s, 3H), 2.02 (d, J = 3.7 Hz, 6H), 1.56 (p, J = 3.1 Hz, 4H). MS (ESI) m/z [M + Na]⁺ =474.4.

### Step 3: Synthesis of G4-4

Using G4-3 (194.0 g, 429.7 mol) as the starting material and following the synthetic method for G1-6, G4-4 (51.2 g, 157.4 mmol) was obtained as a colorless transparent oil in a two-step combined yield of 41.7%. 1H NMR (400 MHz, DMSO-d6) δ 8.44 (t, J = 5.7 Hz, 1H), 7.87 - 7.80 (m, 2H), 7.56 - 7.40 (m, 3H), 4.96 (d, J = 4.4 Hz, 1H), 4.78 (d, J = 6.6 Hz, 1H), 4.73 (d, J = 1.2 Hz, 1H), 4.59 (t, J = 5.7 Hz, 1H), 3.84 (td, J = 6.5, 4.7 Hz, 1H), 3.74 (ddd, J = 17.5, 7.7, 4.2 Hz, 2H), 3.63 (dt, J = 9.0, 5.7 Hz, 1H), 3.52 (ddd, J = 11.5, 5.7, 3.9 Hz, 1H), 3.35 (q, J = 5.7 Hz, 2H), 3.26 (q, J = 6.4 Hz, 2H), 1.54 (dd, J = 6.7, 3.6 Hz, 4H). MS (ESI) m/z [M - H]⁻ =324.4.

### Step 4: Synthesis of G4-5

Using G4-4 (45.0 g, 138.3 mmol) as the starting material and following the synthetic method for G1-7, G4-5 (59.0 g, 103.9 mmol) was obtained as a white solid in a yield of 75.2%. 1H NMR (400 MHz, DMSO-d6) δ 8.42 (t, J = 5.7 Hz, 1H), 7.87 - 7.80 (m, 2H), 7.50 (dd, J = 8.4, 6.0 Hz, 1H), 7.44 (t, J = 7.4 Hz, 2H), 5.04 (d, J = 3.9 Hz, 1H), 4.72 (s, 1H), 4.30 (dd, J = 7.3, 4.4 Hz, 1H), 3.93 - 3.73 (m, 4H), 3.56 (dt, J = 9.3, 6.0 Hz, 1H), 3.33 (d, J = 6.6 Hz, 1H), 3.25 (d, J = 5.9 Hz, 2H), 1.53 (p, J = 6.6 Hz, 4H), 1.06 - 0.82 (m, 28H). MS (ESI) m/z [M - H]⁻ =566.6.

### Step 5: Synthesis of G4-6

Using G4-5 (59.0 g, 103.9 mmol) as the starting material and following the synthetic method for G1-8, G4-6 (41.0 g, 70.5 mmol) was obtained as a wine-red oil in a yield of 67.8%. 1H NMR (400 MHz, DMSO-d6) δ 8.42 (t, J = 5.7 Hz, 1H), 7.86 - 7.79 (m, 2H), 7.51 (t, J = 7.3 Hz, 1H), 7.44 (dd, J = 8.2, 6.5 Hz, 2H), 4.82 (s, 1H), 4.41 (dd, J = 7.5, 4.3 Hz, 1H), 3.91 - 3.83 (m, 1H), 3.83 - 3.74 (m, 2H), 3.57 (dd, J = 8.4, 5.3 Hz, 2H), 3.45 (s, 3H), 3.40 - 3.32 (m, 1H), 3.24 (t, J = 6.1 Hz, 2H), 1.52 (dt, J = 10.1, 5.1 Hz, 4H), 1.09 - 0.86 (m, 28H). MS (ESI) m/z [M - H]⁻ =580.5.

### Step 6: Synthesis of G4-7

Using G4-6 (34.0 g, 58.4 mmol) as the starting material and following the synthetic method for G1-9, G4-7 (22.0 g) was obtained as a pale yellow oil, which was directly used in the next step. 1H NMR (400 MHz, DMSO-d6) δ 8.44 (t, J = 5.7 Hz, 1H), 7.84 (dt, J = 7.0, 1.5 Hz, 2H), 7.56 - 7.48 (m, 1H), 7.45 (dd, J = 8.1, 6.5 Hz, 2H), 4.86 (d, J = 1.7 Hz, 1H), 4.82 (d, J = 6.8 Hz, 1H), 4.62 (s, 1H), 3.96 (td, J = 6.5, 4.8 Hz, 1H), 3.83 - 3.70 (m, 1H), 3.70 - 3.56 (m, 2H), 3.50 (ddd, J = 11.5, 5.7, 4.2 Hz, 1H), 3.44 (dd, J = 4.8, 1.8 Hz, 1H), 3.41 - 3.33 (m, 4H), 3.33 - 3.22 (m, 2H), 1.54 (h, J = 4.7, 3.8 Hz, 4H). MS (ESI) m/z [M - H]⁻ =338.3.

### Step 7: Synthesis of G4-8

Using G4-7 (20.0 g, 58.9 mmol) as the starting material and following the synthetic method for G1-10, G4-8 (23.1 g, 36.0 mmol) was obtained as a pale yellow foamy solid in a yield of 61.0%. 1H NMR (400 MHz, DMSO-d6) δ 8.40 (t, J = 5.7 Hz, 1H), 7.86 - 7.79 (m, 2H), 7.55 - 7.48 (m, 1H), 7.48 - 7.40 (m, 4H), 7.33 - 7.24 (m, 6H), 7.18 (t, J = 7.3 Hz, 1H), 6.87 (d, J = 8.3 Hz, 4H), 4.94 (s, 1H), 4.86 (d, J = 7.3 Hz, 1H), 4.02 (td, J = 7.1, 4.2 Hz, 1H), 3.91 (td, J = 7.2, 6.6, 2.7 Hz, 1H), 3.72 (s, 6H), 3.70 - 3.63 (m, 1H), 3.45 (d, J = 4.5 Hz, 1H), 3.43 - 3.35 (m, 4H), 3.22 (dd, J = 7.9, 4.5 Hz, 2H), 3.10 (dd, J = 10.0, 2.8 Hz, 1H), 2.95 (dd, J = 10.0, 5.8 Hz, 1H), 1.48 (dd, J = 6.6, 3.4 Hz, 4H). MS (ESI) m/z [M - H]⁻ =640.6.

### Step 8: Synthesis of G4-9

Using G4-8 (21.0 g, 32.7 mmol) as the starting material and following the synthetic method for G1-11, a crude product G4-9 (16.5 g) was obtained as a pale yellow solid, which directly used in the next step. 1H NMR (400 MHz, DMSO-d6) δ 7.47 - 7.40 (m, 2H), 7.34 - 7.25 (m, 6H), 7.21 (t, J = 7.1 Hz, 1H), 6.88 (d, J = 8.7 Hz, 4H), 4.93 (s, 1H), 4.88 (s, 1H), 4.00 (dd, J = 7.3, 4.6 Hz, 1H), 3.95 - 3.88 (m, 1H), 3.73 (s, 6H), 3.65 - 3.58 (m, 1H), 3.44 (d, J = 4.5 Hz, 1H), 3.41 - 3.32 (m, 4H), 3.10 (dd, J = 10.0, 2.7 Hz, 1H), 2.94 (dd, J = 9.9, 5.9 Hz, 1H), 2.45 (t, J = 6.9 Hz, 2H), 1.43 (q, J = 7.0 Hz, 2H), 1.26 (ddd, J = 11.1, 8.7, 5.4 Hz, 2H). MS (ESI) m/z [M + Na]⁺=560.5.

### Step 9: Synthesis of G4-10

Using G4-9 (15.0 g, 27.9 mmol) and G1-12 (13.7 g, 30.6 mmol) as starting materials and following the synthetic method for G1-13, G4-10 (19.4 g, 20.1 mmol) was obtained as a pale yellow foamy solid in a yield of 71.9%. 1H NMR (400 MHz, DMSO-d6) δ 7.80 (d, J = 9.2 Hz, 1H), 7.66 (t, J = 5.6 Hz, 1H), 7.46 - 7.39 (m, 2H), 7.34 - 7.25 (m, 6H), 7.25 - 7.17 (m, 1H), 6.91 - 6.84 (m, 4H), 5.22 (d, J = 3.4 Hz, 1H), 4.97 (dd, J = 11.2, 3.4 Hz, 1H), 4.93 (d, J = 1.1 Hz, 1H), 4.86 (d, J = 7.3 Hz, 1H), 4.49 (d, J = 8.6 Hz, 1H), 4.07 - 3.96 (m, 4H), 3.95 - 3.84 (m, 2H), 3.74 (s, 6H), 3.67 - 3.56 (m, 2H), 3.47 - 3.33 (m, 6H), 3.10 (dd, J = 10.0, 2.8 Hz, 1H), 2.96 (td, J = 11.7, 10.2, 6.1 Hz, 3H), 2.10 (s, 3H), 2.03 (t, J = 7.1 Hz, 2H), 1.99 (s, 3H), 1.89 (s, 3H), 1.77 (s, 3H), 1.45 (tq, J = 13.1, 6.6 Hz, 6H), 1.34 (d, J = 14.9 Hz, 2H). MS (ESI) m/z [M - H]⁻=965.8.

### Step 10: Synthesis of YK-GAL-504

Using G4-10 (3.0 g, 3.10 mmol) as the starting material and following the synthetic method for YK-GAL-501, YK-GAL-504 (3.2 g, 2.74 mmol) was obtained as a white foamy solid in a yield of 88.4%. 1H NMR (400 MHz, DMSO-d6) δ 7.80 (d, J = 9.2 Hz, 1H), 7.70 - 7.61 (m, 1H), 7.43 (dd, J = 7.7, 5.3 Hz, 2H), 7.29 (td, J = 9.0, 8.2, 2.8 Hz, 6H), 7.21 (dt, J = 8.2, 3.8 Hz, 1H), 6.87 (dd, J = 8.7, 4.1 Hz, 4H), 5.21 (d, J = 3.4 Hz, 1H), 4.97 (dd, J = 11.4, 3.6 Hz, 2H), 4.49 (d, J = 8.5 Hz, 1H), 4.03 (q, J = 4.9, 3.9 Hz, 5H), 3.87 (dt, J = 11.2, 8.9 Hz, 1H), 3.73 (d, J = 2.1 Hz, 6H), 3.70 - 3.56 (m, 3H), 3.56 - 3.35 (m, 8H), 3.21 (ddd, J = 27.3, 10.4, 2.8 Hz, 1H), 3.02 - 2.85 (m, 4H), 2.75 (t, J = 6.0 Hz, 1H), 2.56 (t, J = 5.9 Hz, 1H), 2.10 (s, 3H), 2.01 (d, J = 12.3 Hz, 5H), 1.89 (s, 3H), 1.77 (s, 3H), 1.45 (hept, J = 7.0 Hz, 6H), 1.33 (dt, J = 10.2, 4.9 Hz, 2H), 1.22 - 1.03 (m, 12H). MS (ESI) m/z [M + H]⁺=1168.3.

### (5) Synthesis of YK-GAL-505

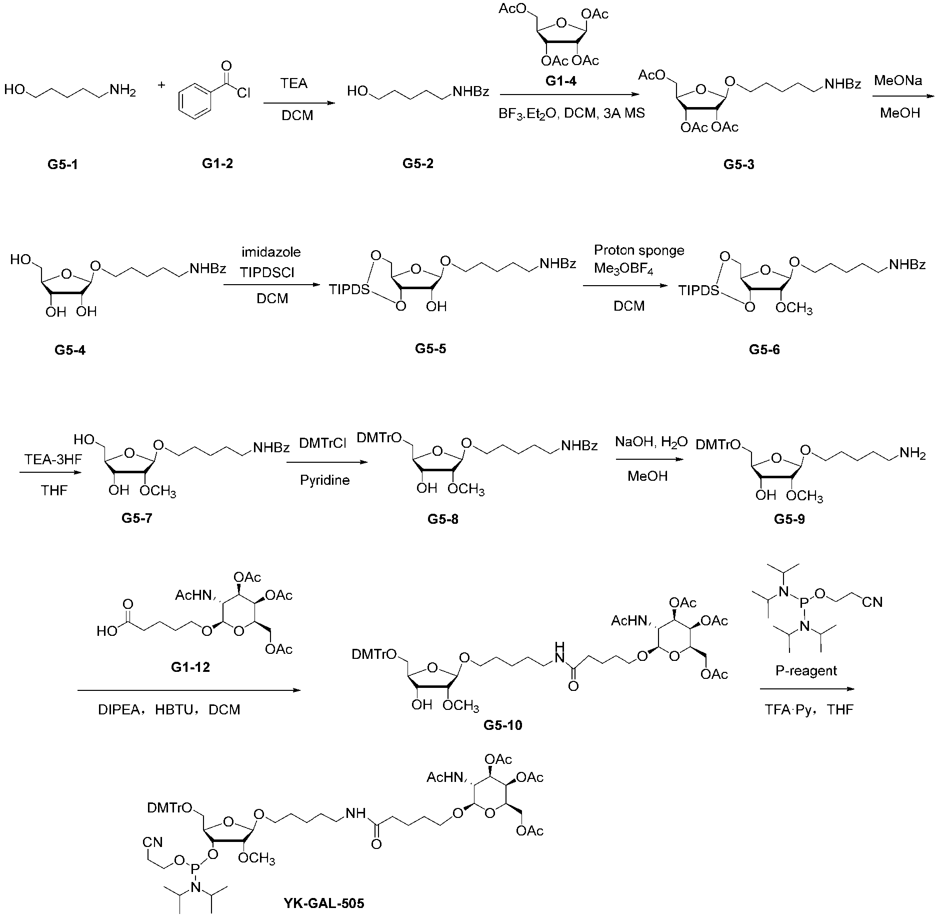

### Step 1: Synthesis of G5-2

Using G5-1 (60.0 g, 581.6 mmol) and G1-2 (81.8 g, 581.9 mmol) as starting materials and following the synthetic method for G1-3, a crude product G5-2 (123.0 g) was obtained as a colorless oil, which was directly used in the next step. 1H NMR (400 MHz, DMSO-d6) δ 8.41 (s, 1H), 7.88 - 7.79 (m, 2H), 7.56 - 7.49 (m, 1H), 7.42 (dd, J = 8.1, 6.5 Hz, 2H), 4.56 (t, J = 5.2 Hz, 1H), 3.34 (td, J = 6.7, 5.6 Hz, 4H), 1.67-1.53 (m, 4H) , 1.34 (m, 2H). MS (ESI) m/z [M - H]⁻ =208.4.

### Step 2: Synthesis of G5-3

Using G1-4 (80.0 g, 251.4 mmol) and G5-2 (78.9 g, 377.1 mmol) as starting materials and following the synthetic method for G1-5, a crude product G5-3 (124.0 g) was obtained as a yellow oil, which was directly used in the next step. 1H NMR (400 MHz, DMSO-d6) δ 8.43 (t, J = 5.7 Hz, 1H), 7.88 - 7.81 (m, 2H), 7.55 - 7.49 (m, 1H), 7.48 (dd, J = 8.1, 6.5 Hz, 2H), 5.16 (dd, J = 6.6, 5.0 Hz, 1H), 5.07 (dd, J = 4.8, 1.2 Hz, 1H), 5.01 (d, J = 1.3 Hz, 1H), 4.32 - 4.17 (m, 2H), 4.05 (ddt, J = 11.0, 7.0, 3.4 Hz, 1H), 3.70 - 3.59 (m, 1H), 3.41 - 3.32 (m, 1H), 3.26 (t, J = 6.0 Hz, 2H), 2.11 (s, 3H), 2.01 (d, J = 3.5 Hz, 6H), 1.54 (p, J = 3.0 Hz, 4H), 1.34 (p, J = 2.0 Hz, 2H). MS (ESI) m/z [M + Na]⁺ =488.3.

### Step 3: Synthesis of G5-4

Using G5-3 (88.0 g, 189.0 mol) as the starting material and following the synthetic method for G1-6, G5-4 (23.0 g, 67.8 mmol) was obtained as a colorless transparent oil in a two-step combined yield of 35.8%. 1H NMR (400 MHz, DMSO-d6) δ 8.44 (t, J = 5.7 Hz, 1H), 7.88 - 7.81 (m, 2H), 7.55 - 7.48 (m, 3H), 4.97 (d, J = 4.6 Hz, 1H), 4.78 (d, J = 6.5 Hz, 1H), 4.75 (d, J = 1.3 Hz, 1H), 4.58 (t, J = 5.7 Hz, 1H), 3.80 (td, J = 6.9, 4.8 Hz, 1H), 3.71 (ddd, J = 17.9, 7.6, 4.2 Hz, 2H), 3.65 (dt, J = 9.0, 5.9 Hz, 1H), 3.51 (ddd, J = 11.2, 5.9, 3.5 Hz, 1H), 3.36 (q, J = 5.7 Hz, 2H), 3.28 (q, J = 6.5 Hz, 2H), 1.54 (p, J = 3.1 Hz, 4H), 1.34 (p, J = 2.1 Hz, 2H). MS (ESI) m/z [M - H]⁻ =338.4.

### Step 4: Synthesis of G5-5

Using G5-4 (20.0 g, 58.9 mmol) as the starting material and following the synthetic method for G1-7, G5-5 (25.1 g, 43.1 mmol) was obtained as a white solid in a yield of 73.2%. 1H NMR (400 MHz, DMSO-d6) δ 8.43 (t, J = 5.7 Hz, 1H), 7.88 - 7.81 (m, 2H), 7.52 (dd, J = 8.4, 6.1 Hz, 1H), 7.43 (t, J = 7.2 Hz, 2H), 5.07 (d, J = 3.9 Hz, 1H), 4.79 (s, 1H), 4.26 (dd, J = 7.4, 4.6 Hz, 1H), 3.90 - 3.77 (m, 4H), 3.58 (dt, J = 9.2, 6.3 Hz, 1H), 3.36 (d, J = 6.5 Hz, 1H), 3.21 (d, J = 5.7 Hz, 2H), 1.53 (p, J = 3.0 Hz, 4H), 1.35 (p, J = 2.0 Hz, 2H), 1.04 - 0.87 (m, 28H). MS (ESI) m/z [M - H]⁻ =580.5.

### Step 5: Synthesis of G5-6

Using G5-5 (24.0 g, 41.2 mmol) as the starting material and following the synthetic method for G1-8, G5-6 (19.4 g, 32.6 mmol) was obtained as a wine-red oil in a yield of 78.9%. 1H NMR (400 MHz, DMSO-d6) δ 8.44 (t, J = 5.7 Hz, 1H), 7.87 - 7.79 (m, 2H), 7.50 (t, J = 7.4 Hz, 1H), 7.47 (dd, J = 8.2, 6.5 Hz, 2H), 4.82 (s, 1H), 4.43 (dd, J = 7.4, 4.5 Hz, 1H), 3.90 - 3.85 (m, 1H), 3.86 - 3.73 (m, 2H), 3.56 (dd, J = 8.4, 5.3 Hz, 2H), 3.47 (s, 3H), 3.41 - 3.34 (m, 1H), 3.25 (t, J = 6.5 Hz, 2H), 1.54 (dt, J = 10.2, 5.1 Hz, 4H), 1.35 (p, J = 2.0 Hz, 2H), 1.09 - 0.86 (m, 28H). ¹HNMR (400 MHz,) *δ* ppm. MS (ESI) m/z [M - H]⁻ =594.5.

### Step 6: Synthesis of G5-7

Using G5-6 (19.0 g, 31.9 mmol) as the starting material and following the synthetic method for G1-9, G5-7 (12.3 g) was obtained as a pale yellow oil, which was directly used in the next step. 1H NMR (400 MHz, DMSO-d6) δ 8.44 (t, J = 5.7 Hz, 1H), 7.85 (dt, J = 7.1, 1.5 Hz, 2H), 7.57 - 7.47 (m, 1H), 7.47 (dd, J = 8.2, 6.5 Hz, 2H), 4.83 (d, J = 1.8 Hz, 1H), 4.82 (d, J = 6.5 Hz, 1H), 4.65 (s, 1H), 3.97 (td, J = 6.5, 4.8 Hz, 1H), 3.88 - 3.71 (m, 1H), 3.69 - 3.55 (m, 2H), 3.51 (ddd, J = 11.2, 5.5, 4.3 Hz, 1H), 3.42 (dd, J = 4.9, 1.8 Hz, 1H), 3.40 - 3.33 (m, 4H), 3.33 - 3.21 (m, 2H), 1.54 (h, J = 4.9, 3.7 Hz, 4H), 1.35 (p, J = 2.1 Hz, 2H). MS (ESI) m/z [M - H]⁻ =352.3.

### Step 7: Synthesis of G5-8

Using G5-7 (12.0 g, 34.0 mmol) as the starting material and following the synthetic method for G1-10, G5-8 (15.3 g, 23.3 mmol) was obtained as a pale yellow foamy solid in a yield of 68.6%. 1H NMR (400 MHz, DMSO-d6) δ 8.42 (t, J = 5.7 Hz, 1H), 7.86 - 7.77 (m, 2H), 7.57 - 7.49 (m, 1H), 7.48 - 7.41 (m, 4H), 7.32 - 7.26 (m, 6H), 7.19 (t, J = 7.3 Hz, 1H), 6.87 (d, J = 8.3 Hz, 4H), 4.95 (s, 1H), 4.89 (d, J = 7.3 Hz, 1H), 4.04 (td, J = 7.1, 4.2 Hz, 1H), 3.90 (td, J = 7.2, 6.6, 2.7 Hz, 1H), 3.72 (s, 6H), 3.71 - 3.64 (m, 1H), 3.45 (d, J = 4.5 Hz, 1H), 3.42 - 3.34 (m, 4H), 3.22 (dd, J = 7.9, 4.5 Hz, 2H), 3.11 (dd, J = 10.0, 2.8 Hz, 1H), 2.96 (dd, J = 10.0, 5.8 Hz, 1H), 1.50 (dd, J = 6.6, 3.4 Hz, 4H) , 1.31 (p, J = 2.1 Hz, 2H). MS (ESI) m/z [M - H]⁻ =654.6.

### Step 8: Synthesis of G5-9

Using G5-8 (13.0 g, 19.8 mmol) as the starting material and following the synthetic method for G1-11, a crude product G3-9 (10.4 g) was obtained as a pale yellow solid, which directly used in the next step. 1H NMR (400 MHz, DMSO-d6) δ 7.48 - 7.40 (m, 2H), 7.33 - 7.22 (m, 6H), 7.20 (t, J = 7.1 Hz, 1H), 6.85 (d, J = 8.7 Hz, 4H), 4.93 (s, 1H), 4.86 (s, 1H), 4.01 (dd, J = 7.3, 4.6 Hz, 1H), 3.96 - 3.88 (m, 1H), 3.73 (s, 6H), 3.64 - 3.59 (m, 1H), 3.45 (d, J = 4.5 Hz, 1H), 3.41 - 3.33 (m, 4H), 3.11 (dd, J = 10.0, 2.7 Hz, 1H), 2.95 (dd, J = 9.9, 5.9 Hz, 1H), 2.43 (t, J = 6.9 Hz, 2H), 1.43 (q, J = 7.0 Hz, 2H), 1.39-1.26 (m, 4H). MS (ESI) m/z [M + Na]⁺ =574.5.

### Step 9: Synthesis of G5-10

Using G5-9 (10.0 g, 18.1 mmol) and G1-12 (8.9 g, 19.9 mmol) as starting materials and following the synthetic method for G1-13, G5-10 (11.9 g, 12.1 mmol) was obtained as a pale yellow foamy solid in a yield of 66.9%. 1H NMR (400 MHz, DMSO-d6) δ 7.80 (d, J = 9.2 Hz, 1H), 7.66 (t, J = 5.6 Hz, 1H), 7.46 - 7.39 (m, 2H), 7.32 - 7.25 (m, 6H), 7.21 (t, J = 7.3 Hz, 1H), 6.87 (d, J = 8.6 Hz, 4H), 5.21 (d, J = 3.4 Hz, 1H), 4.97 (dd, J = 11.2, 3.4 Hz, 1H), 4.92 (s, 1H), 4.86 (d, J = 7.3 Hz, 1H), 4.48 (d, J = 8.5 Hz, 1H), 4.05 - 3.95 (m, 4H), 3.95 - 3.83 (m, 2H), 3.73 (s, 6H), 3.72 - 3.66 (m, 1H), 3.60 (d, J = 9.0 Hz, 1H), 3.44 (d, J = 4.6 Hz, 1H), 3.38 (s, 4H), 3.34 (d, J = 8.8 Hz, 1H), 3.09 (dd, J = 9.9, 2.8 Hz, 1H), 2.95 (dq, J = 12.1, 5.8 Hz, 3H), 2.10 (s, 3H), 2.01 (d, J = 12.5 Hz, 5H), 1.89 (s, 3H), 1.77 (s, 3H), 1.44 (td, J = 14.3, 7.1 Hz, 6H), 1.30 (q, J = 7.2 Hz, 2H), 1.18 (s, 2H). MS (ESI) m/z [M - H] ⁻=979.8.

### Step 10: Synthesis of YK-GAL-505

Using G5-10 (5.0 g, 5.10 mmol) as the starting material and following the synthetic method for YK-GAL-501, YK-GAL-505 (5.0 g, 4.23 mmol) was obtained as a white foamy solid in a yield of 83.3%. 1H NMR (400 MHz, DMSO-d6) δ 7.80 (d, J = 9.2 Hz, 1H), 7.70 - 7.63 (m, 1H), 7.46 - 7.40 (m, 2H), 7.32 - 7.26 (m, 6H), 7.21 (dt, J = 8.2, 3.8 Hz, 1H), 6.87 (dd, J = 8.7, 4.1 Hz, 4H), 5.21 (d, J = 3.4 Hz, 1H), 4.97 (dd, J = 11.4, 3.6 Hz, 2H), 4.50 (d, J = 8.5 Hz, 1H), 4.05 - 3.95 (m, 4H), 3.95 - 3.84 (m, 2H), 3.73 (d, J = 2.1 Hz, 6H), 3.69 - 3.51 (m, 3H), 3.51 - 3.30 (m, 8H), 3.22 (ddd, J = 27.3, 10.4, 2.8 Hz, 1H), 3.09 - 2.87 (m, 4H), 2.74 (t, J = 6.0 Hz, 1H), 2.57 (t, J = 5.9 Hz, 1H), 2.10 (s, 3H), 2.01 (d, J = 12.5 Hz, 5H), 1.89 (s, 3H), 1.77 (s, 3H), 1.44 (td, J = 14.3, 7.1 Hz, 6H), 1.31 (dt, J = 10.2, 4.9 Hz, 4H), 1.23 - 1.03 (m, 12H). MS (ESI) m/z [M + H]⁺=1182.2.

### (6) Synthesis of YK-GAL-325

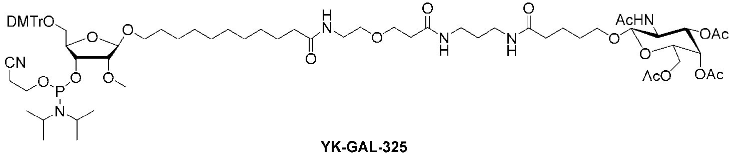

According to the synthesis method for YK-GAL-325 on page 62 of CN116854754B, 417.6 mg of the product was obtained. MS (ESI) m/z [M + H]⁺= 1452.2.

### 2. Synthesis of GalNAc Solid-Phase Support Compound

### (1) Synthesis of YK-GAL-501-SP

### The synthetic route is as follows:

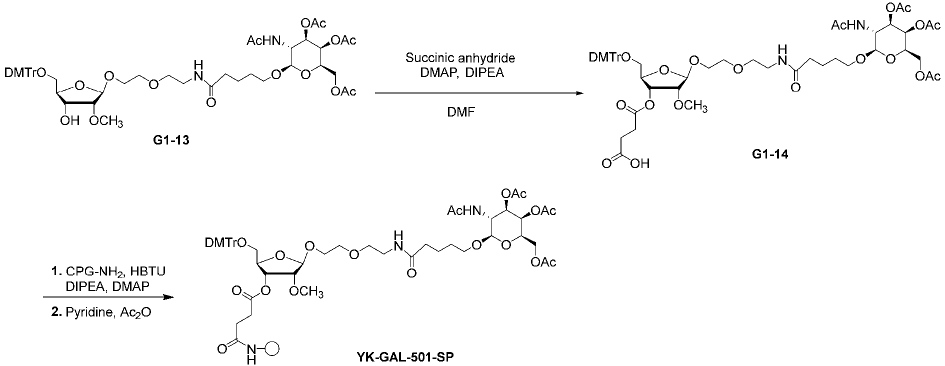

### Step 1: Synthesis of G1-14

G1-13 (200 mg, 204 µmol), 4-dimethylaminopyridine (24.86 mg, 204 µmol), diisopropylethylamine (105.2 mg, 814 µmol), and succinic anhydride (102.1 mg, 1.02 mmol) were dissolved in *N*,*N-*dimethylformamide (2.0 mL). The mixture was stirred at 30°C under nitrogen atmosphere for 16 h. The mixture was purified by preparative chromatography to obtain G1-14 (115.4 mg, 106 µmol) as a white solid in a yield of 52.5%.

### Step 2: Synthesis of YK-GAL-501-SP

G1-14 (50.0 mg, 46.2 µmol), 4-dimethylaminopyridine (5.64 mg, 46.2 µmol), diisopropylethylamine (47.8 mg, 370 µmol), and *O*-(benzotriazol-1-yl)-*N*,*N*,*N'*,*N'-*tetramethyluronium hexafluorophosphate (87.6 mg, 231 µmol) were dissolved in *N,N-*dimethylformamide (10.0 mL), followed by the addition of CPG-NH₂ (800 mg). The mixture was stirred at 40°C for 16 h. The reaction mixture was filtered, and the residue was washed sequentially with methanol and dichloromethane, and dried under vacuum. The residue was then added to a solution of acetic anhydride/pyridine (1:4, 10 mL) and stirred at 40°C for 0.5 h. The mixture was filtered, and the residue was washed sequentially with dichloromethane and methanol, dried under vacuum for 12 h to obtain compound YK-GAL-501-SP (749 mg, loading 32.7 µmol/g) as a white solid.

### (2) Synthesis of Other Solid-Phase Support Compounds

Using G2-10, G3-10, G4-10, and G5-10 as starting materials, respectively, and following the synthetic method for YK-GAL-501-SP, the other GalNAc solid-phase support compounds (YK-GAL-502-SP, YK-GAL-503-SP, YK-GAL-504-SP, and YK-GAL-505-SP) listed in Table 1 were synthesized.

**Table 1 GalNAc solid-phase support compounds**

| Name | Compound Structure |
|---|---|
| YK-GAL-501-SP | 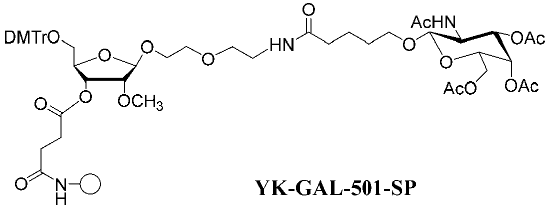 |
| YK-GAL-502-SP | 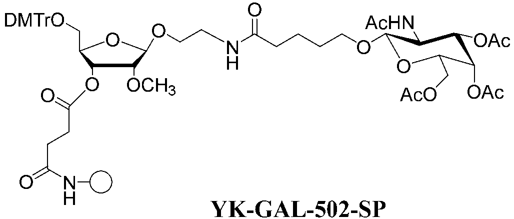 |
| YK-GAL-503-SP | 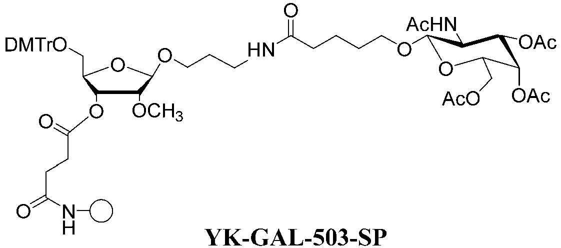 |
| YK-GAL-504-SP | 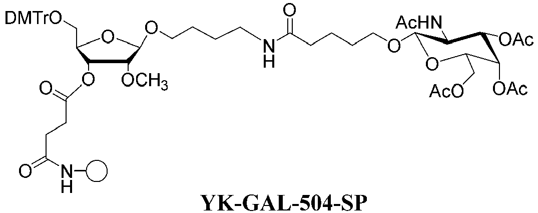 |
| YK-GAL-505-SP | 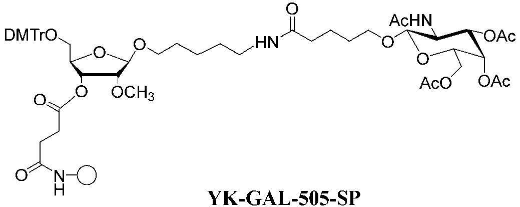 |

### Example 2: Conjugation of GalNAc Compounds with Oligonucleotides

In this example, the same oligonucleotide sequence was used for synthesis. The conjugation of GalNAc compounds with oligonucleotides was performed to obtain GalNAc-conjugated oligonucleotides, wherein the conjugated oligonucleotide sequence was the sequence numbered D579-DV25P. The D579-DV25P sequence is as follows:
Sense strand (D579-DV25P-SS): 5'-Cms-Cms-Um-Um-Um-Um-Cf-Um-Uf-Cf-Uf-Am-Am-Um-Gm-Am-Gf-Um-Cm-Gm-Am-3' (SEQ ID NO: 1),
Antisense strand (D579-DV25P-AS): 5'-UmsEVP-Cfs-Gm-Am-Cm-Uf-Cm-Am-Um-Um-Am-Gm-Am-Af-Gm-Af-Am-Am-Am-Gm-Gms-Ums-Gm -3' (SEQ ID NO: 2).

Herein, A, U, C, and G represent the base composition of the nucleotides; m indicates that the nucleotide immediately to the left of m is 2'-OMe modified; indicates that the nucleotide immediately to the left of is 2'-F modified; s indicates that the two adjacent nucleotides flanking s are connected via a phosphorothioate linkage; EVP indicates that the 5'-end is modified with vinylphosphonate.

The base sequence of the double-stranded oligonucleotide numbered D579-DV25P is:
Sense strand: 5'-CCUUUUCUUCUAAUGAGUCGA-3' (SEQ ID NO:3)
Antisense strand: 5'-UCGACUCAUUAGAAGAAAAGGUG-3' (SEQ ID NO:4)

### 1. Preparation of GalNAc-conjugated siRNA Sense Strand

The GalNAc-conjugated oligonucleotide, hereinafter referred to as the conjugate, was synthesized on a solid-phase support according to the phosphoramidite chemistry method.

When synthesizing conjugates 1-5 (sequence numbers: D579-DV25PG501, D579-DV25PG502, D579-DV25PG503, D579-DV25PG504, and D579-DV25PG505), a universal CPG solid-phase support or the GalNAc solid-phase support compounds synthesized in Example 1 (YK-GAL-501-SP, YK-GAL-502-SP, YK-GAL-503-SP, YK-GAL-504-SP, and YK-GAL-505-SP) were used, and the GalNAc phosphoramidite compounds synthesized in Example 1 (YK-GAL-501, YK-GAL-502, YK-GAL-503, YK-GAL-504, and YK-GAL-505) were used as monomers; When synthesizing conjugate 6 (sequence number: D579-DV25PG325), a universal CPG solid-phase support was used, and the GalNAc phosphoramidite compound YK-GAL-325 synthesized in Example 1 was used as the monomer; When synthesizing conjugate 7 (sequence number: D579-DV25PL96), the purchased CPG-L96(Tianjin WuXi AppTec New Drug Development Co., Ltd, L96 as referenced in claim 10 of US 10465194B2) was used as the solid-phase support, wherein the synthesis scale of the solid-phase support was 1 µmol. These GalNAc compounds were all conjugated to the 3'-end of the oligonucleotide.

### (1) Preparation of reagents and monomers

A monomer solution in acetonitrile (1/20, w/v) was used, with a 0.25 M solution of 5-benzylthiotetrazole in acetonitrile as an activator, a 0.2 M solution of xanthane hydride in acetonitrile/pyridine (1/4, v/v) as a thiolation reagent, a 0.05 M solution of iodine in water/pyridine (1/9, v/v) as an oxidizing reagent, 20% acetic anhydride in acetonitrile (v/v) as capping agent A, 20/30/50 (1-methylimidazole/pyridine/acetonitrile, v/v/v) as capping agent B, 20% diethylamine in acetonitrile (v/v) as a decyanoethylation reagent, and 3% dichloroacetic acid in toluene (v/v) as a DMT-deprotection reagent were prepared. These were loaded into the designated reagent positions of a 192 P model DNA/RNA automated synthesizer.

### (2) Synthesis of crude product

The specified oligonucleotide sequence was input, and the synthesis program was set. After verifying that everything was correct, the oligonucleotide synthesis cycle was initiated. The monomer coupling time was about 1 min, with an oxidation time of about 30-45 sec and a thiolation time of about 2 min. After the cycle was completed, the solid-phase synthesis of the oligonucleotide was finished.

### (3) Deprotection

After the synthesis was completed, the solid-phase support was transferred to a reactor, and the oligonucleotide was cleaved from the solid-phase support using concentrated aqueous ammonia (25-28%) at 50-60°C for 16-24 h. The system was cooled to room temperature, then filtered, and rinsed with a mixed solution of purified water and ethanol. The filtrates were combined and concentrated at low temperature to obtain a crude residue.

### (4) Purification

The deprotected crude residue was dissolved in purified water and subjected to HPLC purification. The product peak solution was collected, and the content was measured using a microplate reader, with the molecular weight confirmed by ESI MS.

In this step, the GalNAc phosphoramidite compound synthesized in Example 1 was conjugated to the 3'-end of the sense strand of siRNA.

When synthesizing conjugates 1-6, the GalNAc phosphoramidite compounds (YK-GAL-501, YK-GAL-502, YK-GAL-503, YK-GAL-504, YK-GAL-505, and YK-GAL-325) synthesized in Example 1 were used as monomers. When a universal CPG solid-phase support was used, the GalNAc phosphoramidite compound was repeated three times in the synthesis sequence; when the GalNAc solid-phase support synthesized in Example 1 was used, the GalNAc phosphoramidite compound was repeated twice in the synthesis sequence, ultimately resulting in conjugates 1-6 each being conjugated with three GalNAc compounds.

### 2. Preparation of siRNA Antisense Strand Without GalNAc Conjugation

The siRNA antisense strand was synthesized according to the method for synthesizing the siRNA sense strand, wherein a universal CPG solid-phase support was used, and the synthesis scale for each antisense strand complementary to the sense strand was 1 µmol.

### 3. Preparation of Conjugated Oligonucleotide

The GalNAc-conjugated siRNA sense strand and complementary antisense strand were hybridized at a ratio of 1:1 based on ultraviolet absorption, heated to 95°C for 3 min, and then cooled to room temperature to form a double strand. The resulting double-strand solution was characterized by HPLC for product purity. After passing the qualification, the content was determined using a microplate reader. The solution was lyophilized to obtain a solid powder, which was stored for future use. The sequences and molecular weights of the obtained GalNAc-conjugated oligonucleotides are shown in Table 2.

**Table 2 GalNAc-conjugated double-stranded siRNA**

| Conjugate | Sequence Number | Sequence Direction 5'-3' | | Theoretical Molecular Weight | Measured Molecular Weight |
|---|---|---|---|---|---|
| Conjugate 1 | D579-DV25PG501 | SS | D579-DV25P-SS-G501 | 8688.1 | 8686.2 |
| | | AS | D579-DV25P-AS | 7833.2 | 7831.8 |
| Conjugate 2 | D579-DV25PG502 | SS | D579-DV25P-SS-G502 | 8555.9 | 8553.9 |
| | | AS | D579-DV25P-AS | 7833.2 | 7831.8 |
| Conjugate 3 | D579-DV25PG503 | SS | D579-DV25P-SS-G503 | 8598.0 | 8596.3 |
| | | AS | D579-DV25P-AS | 7833.2 | 7831.8 |
| Conjugate 4 | D579-DV25PG504 | SS | D579-DV25P-SS-G504 | 8640.1 | 8638.4 |
| | | AS | D579-DV25P-AS | 7833.2 | 7831.8 |
| Conjugate 5 | D579-DV25PG505 | SS | D579-DV25P-SS-G505 | 8682.1 | 8680.5 |
| | | AS | D579-DV25P-AS | 7833.2 | 7831.8 |
| Conjugate 6 | D579-DV25PG325 | SS | D579-DV25P-SS-G325 | 9493.3 | 9491.8 |
| | | AS | D579-DV25P-AS | 7833.2 | 7831.8 |
| Conjugate 7 | D579-DV25PL96 | SS | D579-DV25P-SS-L96 | 8627.4 | 8625.6 |
| | | AS | D579-DV25P-AS | 7833.2 | 7831.8 |

Herein: SS is the sense strand, AS is the antisense strand, and the obtained GalNAc-conjugated oligonucleotide structures are as follows: 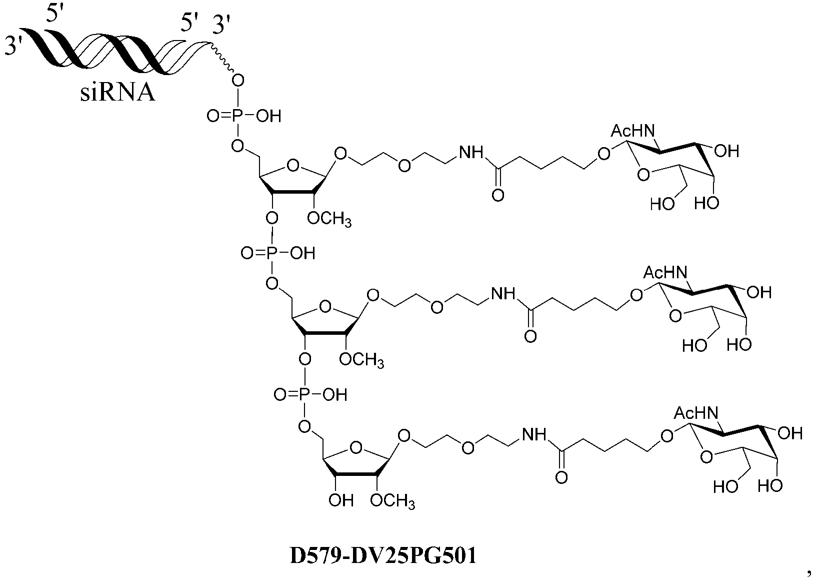 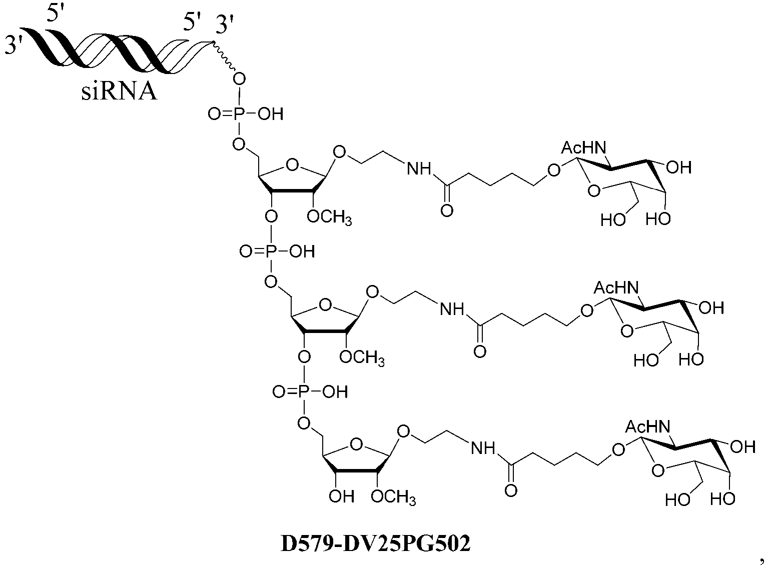 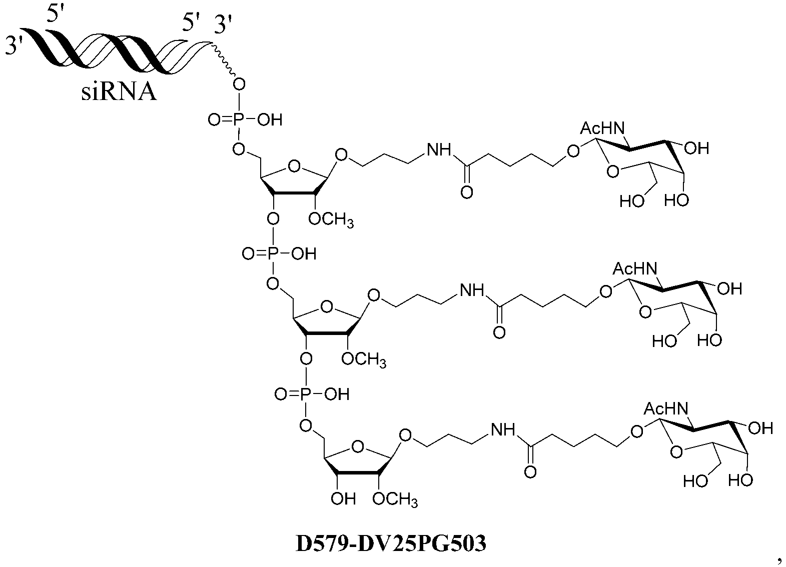 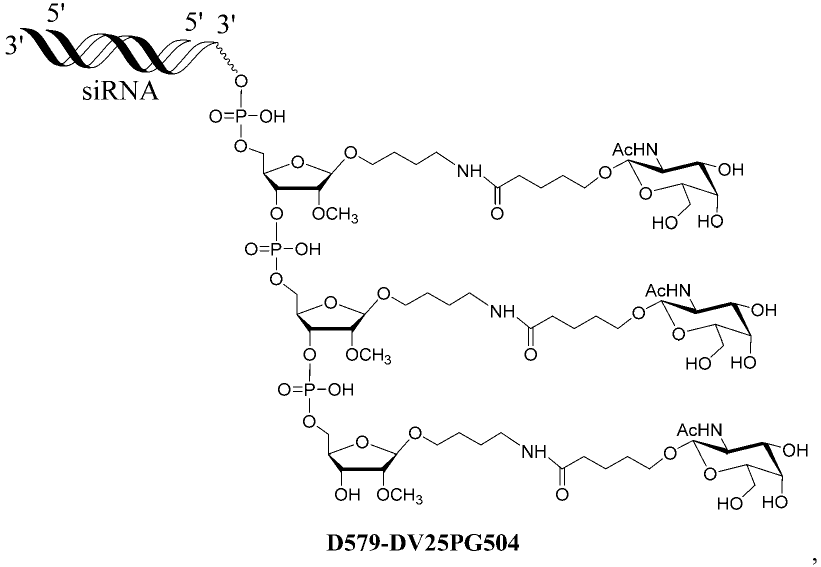 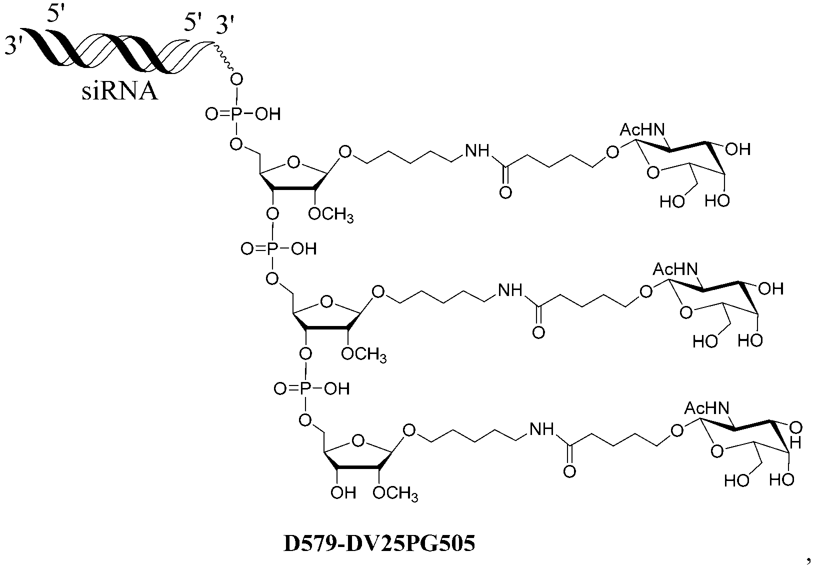 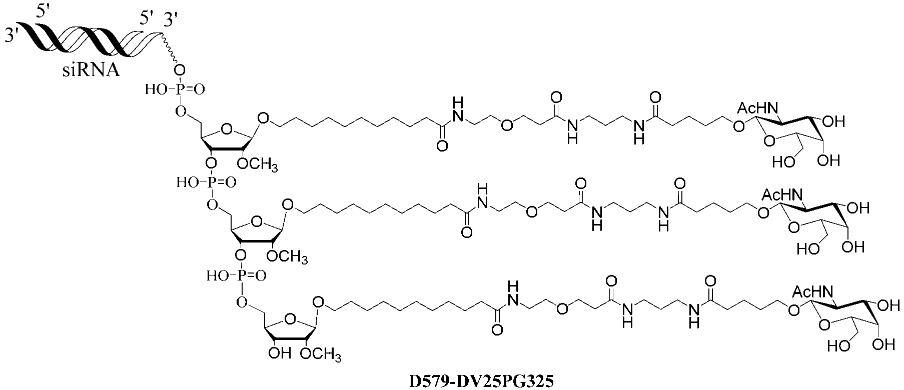
the GalNAc compound in D579-DV25PG325 is YK-GAL-325 (CN116854754B, page 62), 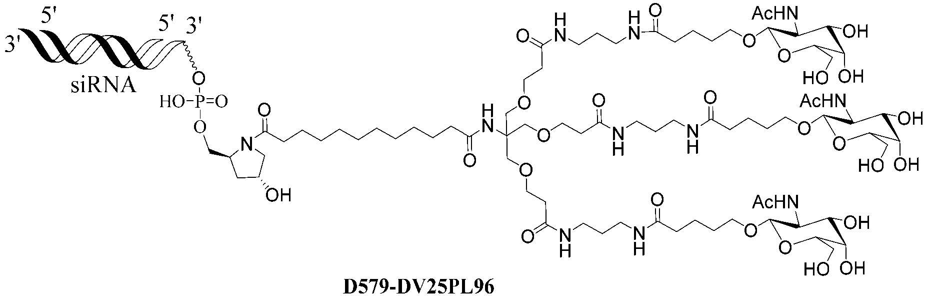
the GalNAc compound in D579-DV25PL96 was L96 (US10465194B2, compound of claim 10).

### Example 3: Inhibitory Effect of GalNAc-Conjugated Oligonucleotides on AGT Protein Expression in Mouse Serum and Liver and Effect on Hepatic AGT mRNA Levels

Angiotensinogen (AGT) protein is a secreted protein that is primarily expressed in the liver in humans. As an upstream protein of the renin-angiotensin-aldosterone system (RAAS), inhibition of AGT expression fundamentally suppresses the blood pressure-elevating effect of the RAAS system, thereby reducing blood pressure.

In this example, transgenic mice expressing the human AGT gene were used to detect the inhibitory effect of the GalNAc-conjugated double-stranded siRNA prepared in Example 2 on the AGT protein in serum at different time points by ELISA, and the inhibitory effect on the AGT mRNA level in the liver was detected.

### 1. Experimental materials

### Test drug:

GalNAc-conjugated oligonucleotides: D579-DV25PL96, D579-DV25PG325, D579-DV25PG501, D579-DV25PG502, D579-DV25PG503, D579-DV25PG504, and D579-DV25PG505.

### Experimental animal information:

Species/strain: hAGT transgenic mice
Grade: SPF
Gender: male
Number: 64
Age: 6-8 weeks
Body weight: 18-28 g
Source: Jiangsu GemPharmatech Co., Ltd.
Production license number: SCXK(Su)2018-0008

### Housing and management:

Housing conditions: After receipt, the experimental animals were housed at Beijing Brightshines Technology Co., Ltd., using license number: SYXK (Beijing) 2022-0025. All procedures were strictly conducted in accordance with the requirements of the Institutional Animal Care and Use Committee (IACUC) to ensure animal welfare. The dimensions of the housing cages were length × width × height = 29.0 cm × 18.5 cm × 13.0 cm; the temperature range was set at 20-26°C, the humidity range was set at 40%-70%, the air exchange rate was no less than 15 complete air changes per hour, with artificial lighting on a 12-hour light/12-hour dark cycle.

The housing environmental conditions were standardized with reference to the National Standard of the People's Republic of China GB14925-2010, and the environment was controlled by a combined air-conditioning unit. The animals were allowed free access to food and water.

### 2. Experimental method

### Dose design and grouping

Definition of experimental dates: The day of administration of vehicle or test drug to animals was defined as day 0.

Grouping and administration: After 3 days of acclimatization, the experimental animals were randomly divided into a negative control group and a test drug group based on serum AGT protein levels, with 8 animals per group. A single subcutaneous injection was administered at a dose of 1 mg/kg, with a dosing volume of 5 mL/kg and a dosing concentration of 0.2 mg/mL. The day of administration was designated as day 0.

Details of animal grouping are shown in Table 3:

**Table 3 Animal grouping information**

| **Group** | **Drug** | **Dose (mg/kg)** | **Route/Frequency of Administration** | **Cycle (Days)** | **Number of Animals** |
|---|---|---|---|---|---|
| Negative Control Group | Vehicle | / | / | 35 | 8 |
| Test Drug Group | siRNA | 1 | Subcutaneous Injection/Once | 35 | 8 |

### Detection indicators

### (1) General observation

Observations were conducted once daily from one week before administration until the end of the experiment.

Observation content: Animals were observed cage-side for mortality or moribund condition, mental status, behavioral activity, fecal characteristics, the supply of feed and water, etc.

Test animals: all animals in the negative control group and the test drug group.

### (2) Expression of AGT protein in serum

Detection time: before administration on day -3 (day -3), 1 week after administration on day 7 (1 w), 2 weeks after administration on day 14 (2 w), 3 weeks after administration on day 21 (3 w), 4 weeks after administration on day 28 (4 w), 5 weeks after administration on day 35 (5 w).

AGT protein level detection method: An ELISA kit was used for detection.

Test animals: all animals in the negative control group and the test drug group.

### (3) Detection of liver mRNA levels

Collection of liver tissue and preparation of homogenate: On Day 35, an appropriate amount of fresh liver tissue was collected from all animals. According to the ratio of tissue weight to RNA lysis buffer (TRIzol) = 100 mg:1 mL, the tissue was rapidly placed into a pre-prepared EP tube containing 1 mL of RNA lysis buffer (TRIzol) to prepare liver homogenate. The homogenate was immediately tested or stored at -80°C. The remaining liver tissue was flash- frozen and stored at -80°C.

RNA extraction:
a. The tissue was placed into a 1.5 mL RNase-free EP tube, and 1 mL of TRIzol reagent was added per 100 mg of tissue, followed by shaking and grinding to prepare a tissue homogenate.
b. Centrifugation was performed at 4°C, 12000 g for 3 min, and 400 µL of the tissue homogenate supernatant was transferred to a 1.5 mL RNase-free EP tube, which was placed on ice. 80 µL of chloroform was added to each tube, followed by vigorous shaking for 15 sec and standing at room temperature for 5 min. Centrifugation was performed at 4°C, 12000 g for 15 min, and 150 µL of the supernatant was transferred to a new EP tube.
c. An equal volume of isopropanol was added, and the liquid in the tube was gently mixed by inverting, allowed to stand at -20°C for 10 min, centrifuged at 4°C (white precipitate was visible), 12000 g for 15 min, and the supernatant was discarded.
d. 1 mL of 75% ethanol was added to gently wash the RNA precipitate, centrifuged at 4°C, 7500 g for 5 min, and the supernatant was aspirated. The rinsing step was repeated once, followed by centrifugation at 7500 g for 5 min at 4°C, and any residual ethanol was thoroughly removed using a micropipette tip.
e. The residual ethanol was air-dried at room temperature for 10 min, and 100 µL of RNase-free ddH₂O was added for dissolution.

RNA concentration detection and reverse transcription: the RNA concentration was detected using a UV-visible spectrophotometer. 2 µL of RNase-free ddH₂O was used as a blank control, and 2 µL of the RNA sample was used for each detection. The sample concentration was recorded. Subsequently, cDNA was synthesized using PrimeScript RT Master Mix according to the instructions. The reverse transcription reaction system was prepared in a 0.2 mL eight-tube strip according to Table 4. The volumes of RNA and sterile nuclease-free water could be adjusted based on the RNA concentration. A 10 µL system could process up to 500 ng of RNA, and the system could be scaled proportionally according to the required amount of RNA.

**Table 4 Reverse transcription system**

| Component | Volume |
|---|---|
| Master Mix | 2.0 µL |
| RNA | 5.0 µL |
| Sterile Nuclease-Free Water | 3.0 µL |

The eight-tube strip was gently flicked to mix the system. The mixture was briefly centrifuged using the Short function of the centrifuge, followed by a reverse transcription reaction in the PCR instrument with the following program: 37°C for 15 min, 85°C for 5 s, and hold at 4°C.

Realtime-qPCR: qPCR was performed using TB Green^{®} Premix Ex Taq^{™} (Tli RNaseH Plus), Bulk. A 10 µL reaction system was prepared in a 96-well plate according to Table 4, and the cDNA of GAPDH and AGT was detected by qPCR, with GAPDH used as the internal reference gene. qPCR was conducted in a 96-well plate, with each well containing 10 µL of reaction system. For each sample, three wells were used for GAPDH primers and three wells for each pair of target gene primers. When the number of samples was excessive and required the distribution of one biological replicate across multiple 96-well plates for detection, it was ensured that each group of samples was amplified simultaneously on the same 96-well plate, and each 96-well plate included control group samples. The qPCR reaction program was set as follows: heating at 95°C for 30 s, followed by a cyclic mode consisting of heating at 95°C for 5 s and then at 60°C for 34 s, for a total of 40 cycles; the template was heated at 95°C for 15 s, at 60°C for 1 min, and then at 95°C for 15 s for melting curve analysis.

**Table 5 qPCR reaction system**

| **Component** | **Volume/µL** |
|---|---|
| 2 × Master Mix | 5.00 |
| Upstream primer (10 µM) | 0.20 |
| Downstream primer (10 µM) | 0.20 |
| cDNA | 1.00 |
| ROX | 0.04 |
| ddH₂O | 3.56 |

### (4) Data processing and statistical analysis

Experimental data were expressed as mean ± standard deviation (Mean ± SD), and data analysis was performed using GraphPad Prism 8.3 software. Statistical analysis was performed using two-way analysis of variance (ANOVA) and post-hoc tests. Homogeneity of variance was assessed using LSD test, whereas heterogeneity of variance was assessed using Dunnett T3 test. A value of P < 0.05 was considered statistically significant.

### 3. Experimental results

The specific experimental results are shown in Tables 6-1, 6-2, and 7.

### (1) Inhibition rate of AGT protein levels in serum

**Table 6-1 Inhibition rates of AGT protein levels in serum by different GalNAc-conjugated oligonucleotides**

| Group | hAGT Baseline | | D7 Inhibition Rate (%) | | D14 Inhibition Rate (%) | | D21 Inhibition Rate (%) | |
|---|---|---|---|---|---|---|---|---|
| | Mean | Standard deviation | Mean | Standard deviation | Mean | Standard deviation | Mean | Standard deviation |
| PBS (Blank) | 16.19 | 1.12 | -1.37 | 6.48 | 8.32 | 14.20 | -12.78 | 10.03 |
| D579-DV25PG325 | 14.86 | 1.53 | 60.06 | 2.77 | 71.43 | 1.83 | 67.26 | 1.88 |
| D579-DV25PL96 | 15.06 | 0.99 | 57.02 | 3.96 | 69.06 | 2.94 | 64.30 | 2.95 |
| D579-DV25PG501 | 15.07 | 1.34 | 66.21 | 1.93 | 75.63 | 2.57 | 73.06 | 3.13 |
| D579-DV25PG502 | 15.02 | 1.14 | 69.24 | 4.33 | 76.61 | 1.99 | 75.47 | 2.40 |
| D579-DV25PG503 | 14.80 | 1.08 | 67.91 | 5.23 | 77.15 | 2.20 | 73.25 | 5.02 |
| D579-DV25PG504 | 14.87 | 1.05 | 67.50 | 3.57 | 77.35 | 2.25 | 73.87 | 2.64 |
| D579-DV25PG505 | 14.90 | 1.72 | 63.87 | 5.61 | 74.86 | 5.06 | 71.08 | 5.18 |

**Table 6-2 Inhibition rates of AGT protein levels in serum by different GalNAc-conjugated oligonucleotides**

| Group | hAGT Baseline | | D28 Inhibition Rate (%) | | D35 Inhibition Rate (%) | |
|---|---|---|---|---|---|---|
| | Mean | Standard deviation | Mean | Standard deviation | Mean | Standard deviation |
| PBS (Blank) | 16.19 | 1.12 | -3.72 | 8.06 | -7.16 | 9.97 |
| D579-DV25PG325 | 14.86 | 1.53 | 67.13 | 3.24 | 63.04 | 3.85 |
| D579-DV25PL96 | 15.06 | 0.99 | 66.25 | 3.90 | 57.00 | 9.24 |
| D579-DV25PG501 | 15.07 | 1.34 | 75.24 | 4.53 | 67.98 | 3.98 |
| D579-DV25PG502 | 15.02 | 1.14 | 72.83 | 2.78 | 69.50 | 2.68 |
| D579-DV25PG503 | 14.80 | 1.08 | 74.12 | 3.96 | 69.50 | 6.31 |
| D579-DV25PG504 | 14.87 | 1.05 | 75.09 | 2.57 | 71.48 | 3.53 |
| D579-DV25PG505 | 14.90 | 1.72 | 72.09 | 3.52 | 67.31 | 6.53 |

As shown in Table 6, these GalNAc-conjugated oligonucleotides can continuously and significantly inhibit the AGT protein level in mouse serum. For example, the inhibition rates of D579-DV25PG503 reached 67.91%, 77.15%, 73.25%, 74.12%, and 69.50% on day 7, day 14, day 21, day 28, and day 35, respectively. The experimental results indicate that the oligonucleotide sequence D579-DV25P conjugated with the GalNAc compound of the present disclosure can be efficiently delivered to the animal liver and significantly inhibit AGT gene expression.

Compared with the siRNA conjugates prepared from GalNAc compounds **YK-GAL-325** and **L96** in the prior art, the GalNAc-conjugated oligonucleotides prepared from the GalNAc compounds of the present disclosure significantly improve the inhibition rate on the AGT protein level in serum. For example, the inhibition rate of the D579-DV25PG502 group was increased by 12.22% compared to that of the D579-DV25PL96 group on day 7, while the inhibition rate of the D579-DV25PG504 group was increased by 14.48% compared to that of the D579-DV25PL96 group on day 35.

### (2) Inhibition rate of AGT mRNA levels in liver

**Table 7 Inhibition rate of AGT mRNA levels in liver of hAGT transgenic mice after 35 days of administration**

| Group | PBS (blan k) | D579-DV25PG 325 | D579-DV25P L96 | D579-DV25PG 501 | D579-DV25PG 502 | D579-DV25PG 503 | D579-DV25PG 504 | D579-DV25PG 505 |
|---|---|---|---|---|---|---|---|---|
| Inhibition Rate (%) | 100. 00 | 66.25 | 60.94 | 71.29 | 72.71 | 73.19 | 74.64 | 71.16 |
| Standard Deviation | 0.05 | 0.03 | 0.11 | 0.04 | 0.02 | 0.05 | 0.04 | 0.05 |

As shown in Table 7, these GalNAc-conjugated oligonucleotides can continuously and significantly inhibit the AGT mRNA level in mouse liver, with the D579-DV25PG504 group exhibiting the highest inhibition rate of 74.64%. The experimental results indicate that the oligonucleotide sequence D579-DV25P conjugated with the GalNAc compound of the present disclosure can be efficiently delivered to the animal liver and significantly inhibit the AGT mRNA level in the liver.

Compared with the siRNA conjugates prepared from GalNAc compounds **YK-GAL-325** and **L96** in the prior art, the GalNAc-conjugated oligonucleotides prepared from the GalNAc compounds of the present disclosure significantly improve the inhibition rate on the AGT mRNA level in mouse liver. For example, the inhibition rate of the D579-DV25PG504 group was increased by 8.39% and 13.70% compared to that of the D579-DV25PG325 group and the D579-DV25PL96 group, respectively.

Compared with the GalNAc compounds **YK-GAL-325** and **L96** in the prior art, the oligonucleotide sequence D579-DV25P conjugated with the GalNAc compounds of the present disclosure exhibit significantly improved efficiency in delivering the oligonucleotide to the liver compared to conjugation with **YK-GAL-325** and **L96,** indicating that the efficiency of oligonucleotide delivery cannot be simply predicted based on the chemical structure of the GalNAc compound.

The present disclosure designs a series of novel GalNAc compounds, such as **YK-GAL-501, YK-GAL-502, YK-GAL-503, YK-GAL-504,** and **YK-GAL-505.** The GalNAc-conjugated oligonucleotides prepared therefrom can achieve efficient liver-targeted delivery, with significantly enhanced activity compared to representative GalNAc compounds in the prior art.
1. The GalNAc compounds of the present disclosure are entirely new GalNAc compounds with chemical structures completely different from those of the GalNAc compounds in the prior art. The GalNAc compounds designed in the present disclosure introduce a ribose ring structure in the linker arm and introduce an oxygen or sulfur atom at the 1'-position of the ribose ring.
2. The GalNAc compounds designed in the present disclosure are conjugated with the oligonucleotide sequence D579-DV25P, which can efficiently deliver the oligonucleotide to the animal liver and can continuously and significantly inhibit the expression of AGT protein in serum and the mRNA level in the liver. For example, the inhibition rate of D579-DV25PG501 on AGT protein in serum reached 66.21%, 75.63%, and 75.24% on day 7, day 14, and day 28, respectively; after 35 days of administration, the inhibition rate of AGT mRNA levels in the liver reached 71.29%.
3. Compared with the GalNAc compounds (e.g., **L96** and **YK-GAL-325)** in the prior art, the GalNAc-conjugated oligonucleotides prepared from the GalNAc compounds designed in the present disclosure exhibit significantly enhanced inhibition rates of AGT protein expression in mouse serum and significantly enhanced inhibition rates of AGT mRNA levels in mouse liver.
   For example, compared with the **L96**-conjugated oligonucleotide D579-DV25PL96, the **YK-GAL-503**-conjugated oligonucleotide D579-DV25PG503 show a 10.89% increase in the inhibition rate of AGT protein expression in mouse serum on day 7 and a 12.25% increase in the inhibition rate of AGT mRNA level in mouse liver on day 35. This indicates that the GalNAc compounds designed in the present disclosure can significantly improve the delivery efficiency, increase the bioavailability of the drug and improve the pharmacokinetic properties of the drug, thereby exerting better drug efficacy.
4. The present disclosure reveals that GalNAc-conjugated oligonucleotides prepared from structurally similar yet distinct GalNAc compounds are highly likely to exhibit significant differences in the inhibition rates of AGT protein expression in mouse serum and AGT mRNA levels in mouse liver, indicating that it is difficult to accurately predict the delivery efficiency of oligonucleotides based on the chemical structure of the GalNAc compounds.

For example, compared with **L96, YK-GAL-503** only changes the prolinol structure in the backbone of **L96** to a ribose ring structure, while the other structures are exactly the same. However, compared with the **L96**-conjugated oligonucleotide D579-DV25PL96, the **YK-GAL-503**-conjugated oligonucleotide D579-DV25PG503 show a 10.89% increase in the inhibition rate of AGT protein expression in mouse serum after 7 days of administration and a 12.25% increase in the inhibition rate of AGT mRNA level in mouse liver after 35 days. Compared with **YK-GAL-325, YK-GAL-503** only involves shortening the linker between the ribose ring and GalNAc in **YK-GAL-325,** while the other structures are exactly the same. However, compared with the **YK-GAL-325-**conjugated oligonucleotide, the **YK-GAL-503-**conjugated oligonucleotide show a 7.85% increase in the inhibition rate of AGT protein expression in mouse serum after 7 days of administration and a 6.94% increase in the inhibition rate of AGT mRNA level in mouse liver after 35 days.

Although the present disclosure is illustrated by the preceding specific examples, it should not be construed as being limited thereto. Instead, the present disclosure encompasses the general aspects previously disclosed and allows for various modifications and embodiments without departing from the spirit and scope of the present disclosure.
SEQ ID NO:1
   Sense strand (D579-DV25P-SS): 5'-Cms-Cms-Um-Um-Um-Um-Cf-Um-Uf-Cf-Uf-Am-Am-Um-Gm-Am-Gf-Um-Cm-Gm-Am-3'.
SEQ ID NO:2
   Antisense strand (D579-DV25P-AS): 5'-UmsEVP-Cfs-Gm-Am-Cm-Uf-Cm-Am-Um-Um-Am-Gm-Am-Af-Gm-Af-Am-Am-Am-Gm-Gms-Ums-Gm -3'.
SEQ ID NO:3:
   Sense strand: 5'-CCUUUUCUUCUAAUGAGUCGA-3'.
SEQ ID NO:4
   Antisense strand: 5'-UCGACUCAUUAGAAGAAAAGGUG-3'.

## Claims

1. A compound of formula (I) or a pharmaceutically acceptable salt thereof: 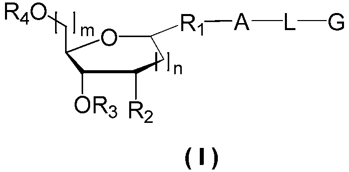
R₁ is O or S;
R₂ is H, C₁₋₆ alkyl, C₁₋₆ alkoxy, or halogen;
R₃ is H, a hydroxyl protecting group, a phosphorus-containing reactive group, - CO(CH₂)ₓCONH-○, or -CO(CH₂)ₓCOOH, wherein x is an integer from 1 to 10, ○ is controlled pore glass or polystyrene;
R₄ is H or a hydroxyl protecting group;
A is -(CH₂)ₐ-, -(CH₂CH₂OCH₂CH₂)_{b}-, or -(CH₂OCH₂)_{c}-, wherein a is an integer from 1 to 10; b is an integer from 1 to 5; c is an integer from 1 to 7;
L is -CONH- or -NHCO-;
G is 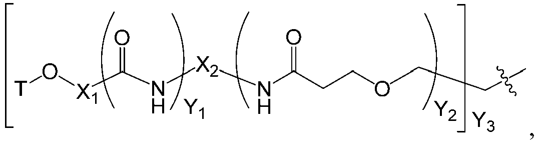
wherein
T is the following group in which all hydroxyl groups are protected with acyl groups: *N-*acetyl-galactosamine, galactose, galactosamine, *N*-formal-galactosamine, *N*-propionyl-galactosamine, or *N*-butyryl-galactosamine;
X₁ is -(CH₂)_{f}- or -(CH₂CH₂O)_{f}CH₂-, wherein f is an integer from 1 to 5;
X₂ is -(CH₂)_{g}-, wherein g is an integer from 1 to 6;
Y₁ is 0 or 1;
Y₂ is 0, 1, or 2;
Y₃ is 1, 2, or 3;
m is an integer from 0 to 4;
n is an integer from 0 to 4.

2. The compound or the pharmaceutically acceptable salt thereof according to claim 1, wherein R₃ is a hydroxyl protecting group, preferably acyl, silyl, trityl, 4-methoxytrityl, or 4,4'-dimethoxytrityl, more preferably acetyl, 1,1,3,3-tetraisopropyldisiloxanyl, *tert*butyldimethylsilyl, dimethylphenylsilyl, or 4,4'-dimethoxytrityl;
alternatively, R₃ is a phosphorus-containing reactive group, such as 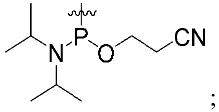
or, R₄ is a hydroxyl protecting group, preferably trityl, 4-methoxytrityl, or 4,4'-dimethoxytrityl;
or, G is 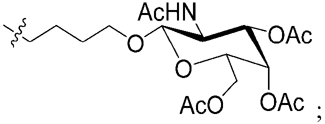
or, the compound or the pharmaceutically acceptable salt thereof is capable of binding to an asialoglycoprotein receptor.

3. The compound or the pharmaceutically acceptable salt thereof according to claim 1, wherein the compound or the pharmaceutically acceptable salt thereof satisfies one or more of the following conditions:
(1) a is an integer from 2 to 7;
(2) b is an integer from 1 to 3;
(3) the acyl group is acetyl or benzoyl, for example, acetyl;
(4) m is 0, 1, or 2;
(5) n is 0, 1, or 2;
(6) R₃ is a phosphorus-containing reactive group or -CO(CH₂)ₓCONH-○, wherein ○ is controlled pore glass or polystyrene, preferably, x is 2.

4. The compound or the pharmaceutically acceptable salt thereof according to claim 1, wherein the compound or the pharmaceutically acceptable salt thereof satisfies one or more of the following conditions:
(1) A is -CH₂CH₂OCH₂CH₂-, -(CH₂)₂-, -(CH₂)₃-, -(CH₂)₄-, or -(CH₂)₅-;
(2) R₁ is O;
(3) R₁ is in *α* configuration or *β* configuration;
(4) R₂ is -OCH₃;
(5) R₃ is -CO(CH₂)₂CONH-○ or -CO(CH₂)₂COOH, wherein ○ is controlled pore glass;
(6) R₄ is 4,4'-dimethoxytrityl;
(7) T is *N*-acetyl-galactosamine in which all hydroxyl groups are protected with acyl groups;
(8) X₁ is -(CH₂)_{f}-, wherein f is 1;
(9) g is 2;
(10) Y₁ is 0;
(11) Y₂ is 0;
(12) m is 1;
(13) n is 0;
(14) L is -NHCO-;
(15) Y₃ is 1.

5. The compound or the pharmaceutically acceptable salt thereof according to claim 1, wherein the compound is any one of the following compounds: 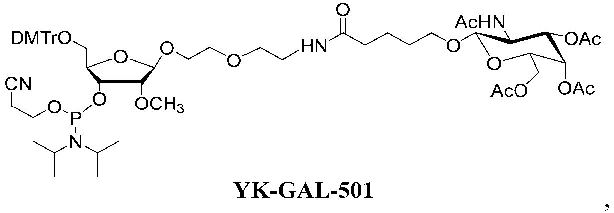 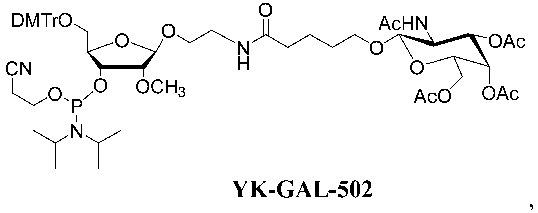 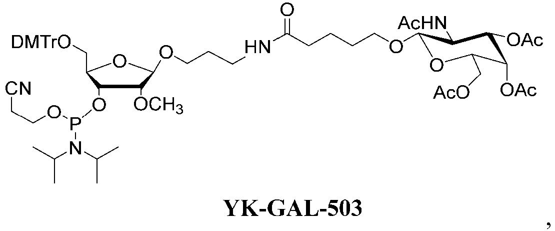 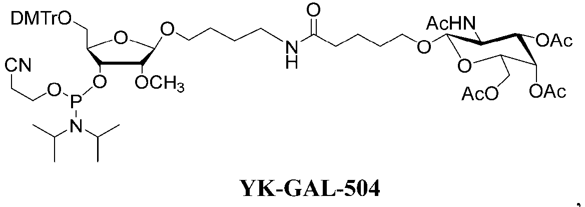 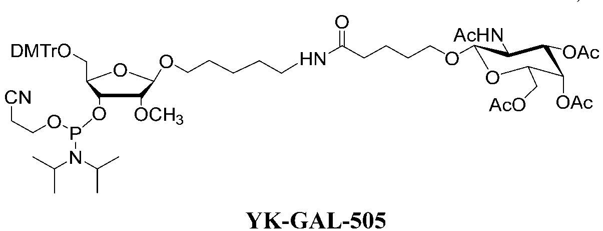 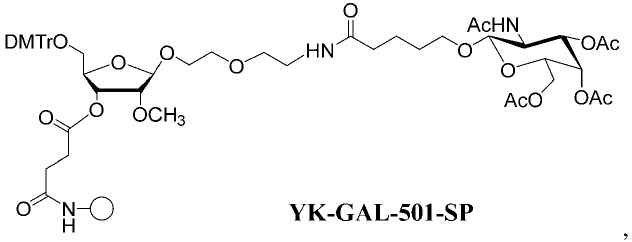 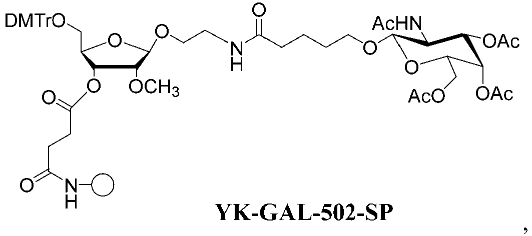 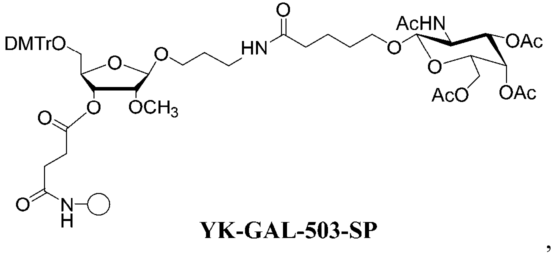 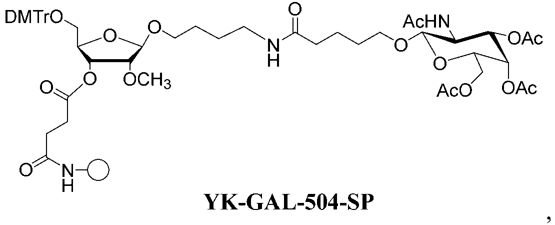 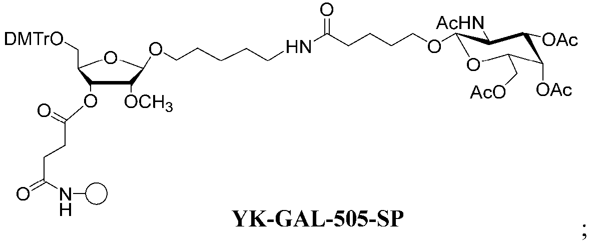 ○ is controlled pore glass.

6. A conjugate comprising an oligonucleotide and a GalNAc moiety, wherein the oligonucleotide and GalNAc are linked via a phosphoester group or a phosphorothioate group; the GalNAc moiety is formed by one or more GalNAc molecules linked via a phosphoester group or a phosphorothioate group;
wherein the GalNAc molecule is a compound of formula I-2 or a pharmaceutically acceptable salt thereof; 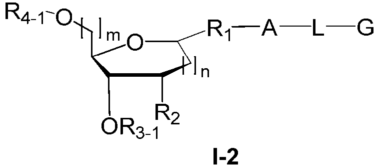
wherein R₃₋₁ is a linking bond, and R₄ is H;
alternatively, R₄₋₁ is a linking bond, and R₃₋₁ is H;
G is 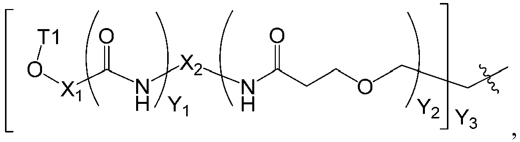
T1 is N-acetyl-galactosamine, galactose, galactosamine, *N*-formal-galactosamine, *N-*propionyl-galactosamine, or *N*-butyryl-galactosamine, preferably *N*-acetyl-galactosamine;
Y₃, Y₂, X₂, Y₁, X₁, m, n, R₂, R₁, A, and L are as defined in any one of claims 1 to 5.

7. The conjugate according to claim 6, wherein the oligonucleotide comprises a non-thio oligonucleotide and a thio oligonucleotide;
or, the oligonucleotide modulates the expression of a target gene.

8. The conjugate according to claim 7, wherein the non-thio oligonucleotide and the GalNAc moiety are linked via a phosphoester bond;
or the thio oligonucleotide and the GalNAc moiety are linked via a phosphorothioate bond.

9. The conjugate according to claim 6, wherein the conjugate has a structure shown below: 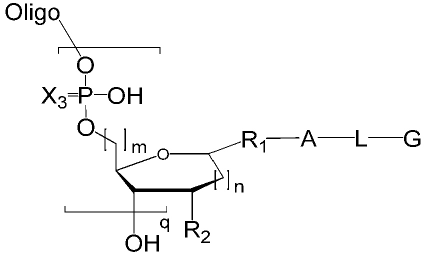 or 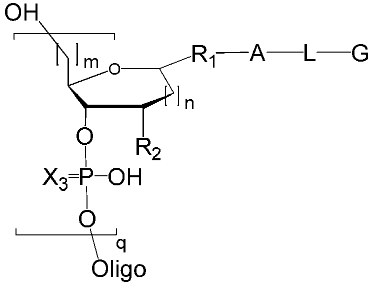 wherein Oligo represents an oligonucleotide, X₃ is O or S, q is 1, 2, or 3, and R₁, R₂, A, L, G, m, and n are as defined in claim 6.

10. The conjugate according to claim 6, wherein the oligonucleotide comprises a small interfering nucleotide, DNA, microRNA, small activating RNA, small guide RNA, transfer RNA, antisense nucleotide, or aptamer, preferably an antisense nucleotide or a small interfering nucleotide; more preferably, each nucleotide in the antisense nucleotide or small interfering nucleotide is independently a modified or unmodified nucleotide.

11. The conjugate according to claim 6, wherein the conjugate has the following structure:

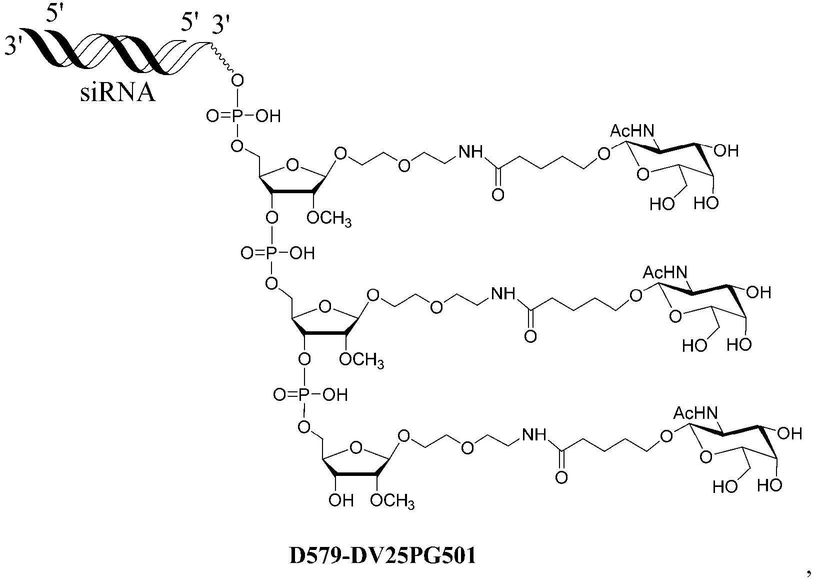

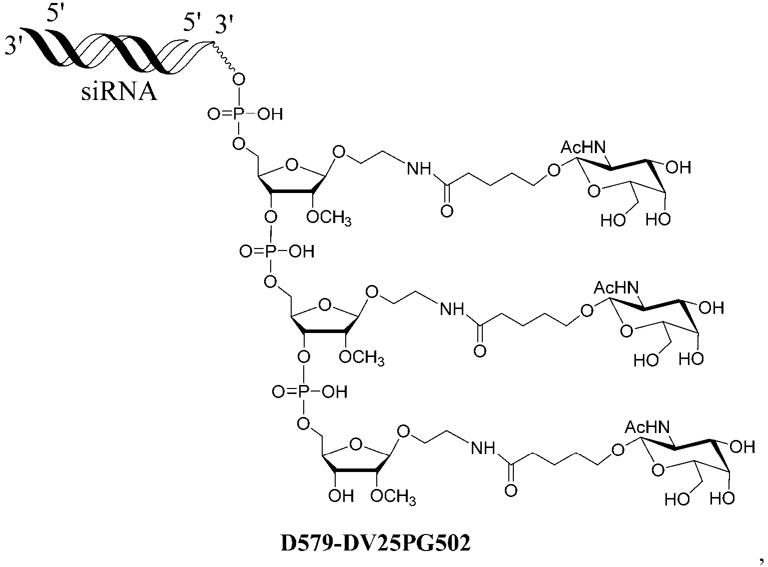

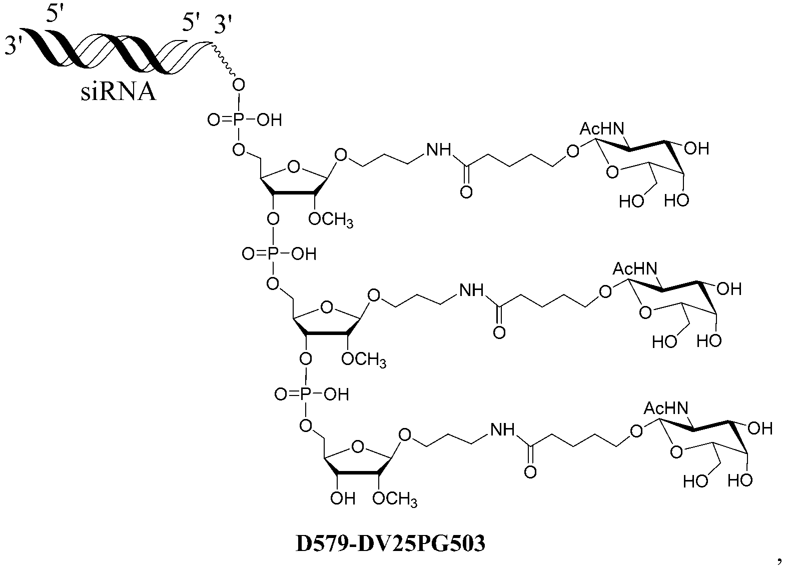

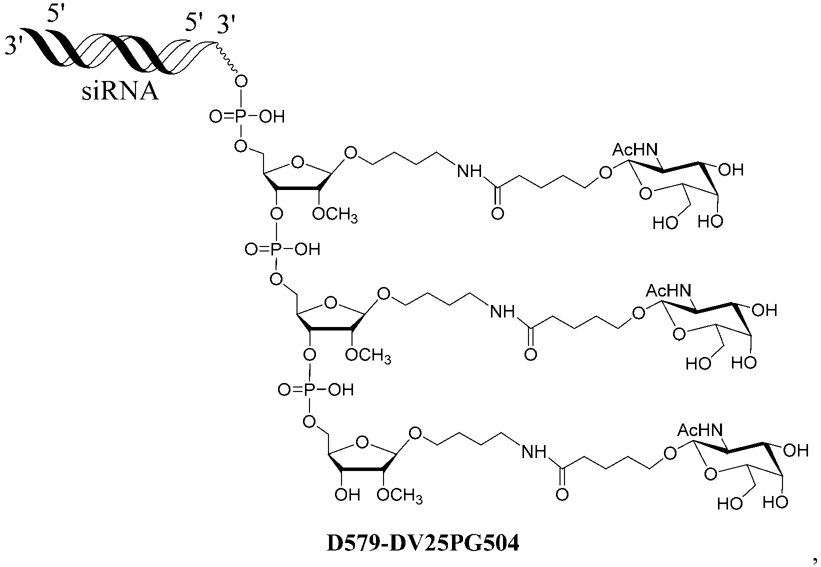

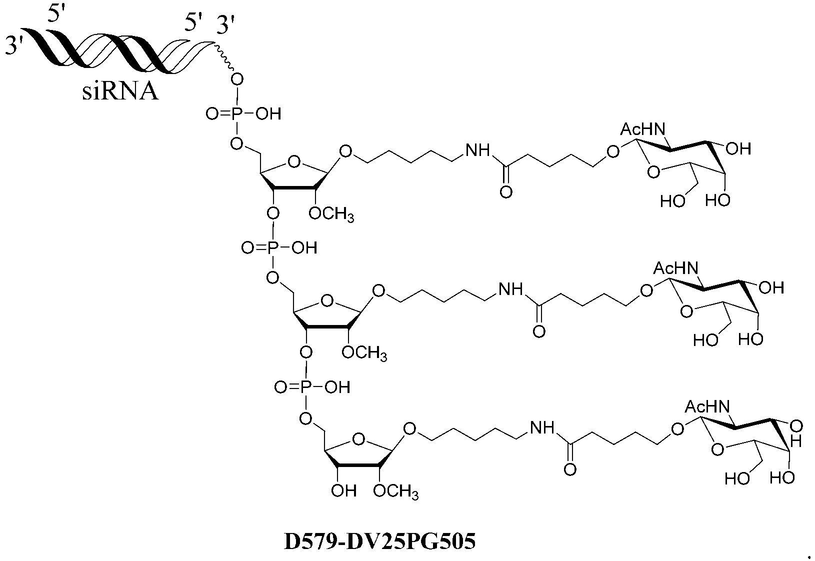

12. A pharmaceutical composition comprising the conjugate according to any one of claims 6 to 11 and at least one pharmaceutically acceptable excipient.

13. The conjugate according to any one of claims 6 to 11 or a pharmaceutically acceptable salt thereof, or the pharmaceutical composition according to claim 12 for use in treating and/or preventing a pathological condition or disease caused by expression of a specific gene in liver tissue or in a virus; preferably, the specific gene is selected from the group consisting of a hepatitis B virus gene, a proprotein convertase subtilisin/kexin type 9 gene, a coagulation factor gene, a lipoprotein(a) gene, an angiopoietin-like protein 3 gene, an angiotensinogen gene, and an apolipoprotein C3 gene.

14. The conjugate or a pharmaceutically acceptable salt thereof, or the pharmaceutical composition for use according to claim 13, wherein the disease is selected from the group consisting of chronic liver disease, hepatitis, liver fibrosis, liver proliferative disease, and cardiovascular and cerebrovascular diseases; preferably, the cardiovascular and cerebrovascular diseases are hypercholesterolemia, hypertriglyceridemia, atherosclerosis, or coagulation dysfunction.

15. A kit comprising the conjugate according to any one of claims 6 to 11.
